# EUROPEAN PATENT APPLICATION

(11) **EP 4 792 836 A1**
(43) Date of publication of application: **19.08.2026**
(21) Application number: 24888097.3
(22) Date of filing: 08.11.2024
(51) Int. Cl.: A61K 48/00, A61K 38/17, A61P 35/00, C12N 15/85, C12N 15/64, C12N 15/12, C12N 15/55

(54) **MULTI-GENE NANOCARRIER, PREPARATION METHOD THEREFOR, APPLICATION THEREOF, AND ANTI-CANCER DRUG**

(30) Priority: 10.11.2023 CN 202311497169
(71) Applicant: Cosychem Biotechnology (TIANJIN) Co., Ltd., Tianjin 300457 (CN)
(72) Inventor: XI, Zhen, Tianjin 300071 (CN); MA, Dejun, Tianjin 300071 (CN); LU, Liqing, Tianjin 300071 (CN)
(74) Representative: reuteler & cie SA
(86) International application number: PCT/CN2024/130905
(87) International publication number: WO 2025/098486

(57) **Abstract**

Provided are a multi-gene nanocarrier, a preparation method therefor, an application thereof, and an anti-cancer drug. The multi-gene nanocarrier comprises a three-branch central ligand and gene combination sequences covalently connected on each branch of the three-branch central ligand, the gene combination sequences on each branch all being the same; in the direction from the three-branch central ligand outward, the gene combination sequences contain at least two tumor suppressor genes selected in order from among TP53, **BIM,** and PTEN. The provided multi-gene nanocarrier has excellent anti-cancer activity and biological safety.

## Description

### CROSS-REFERENCE TO RELATED APPLICATIONS

The application claims priority to the China Application No. "202311497169.8", filed on November 10, 2023, the content of which is specifically and entirely incorporated herein by reference.

### FIELD

The present disclosure relates to the field of biomedicine, in particular to a multi-gene nanovector, a preparation method therefor, a use thereof, and an anti-cancer drug.

### BACKGROUND

A large amount of omics data from databases such as The Cancer Genome Atlas (TCGA), the International Cancer Genome Consortium (ICGC), and the Pan-Cancer Analysis of Whole Genomes (PCAWG) indicate that in all known cancer types, oncogenic driver genes are prone to gene mutations, it is particularly evident in tumor suppressor genes and proto-oncogenes such as *TP53*, *c-Myc*, *PIK3CA*, *KRAS*, *PTEN*, *CDKN2A*, *EGFR*, *BIM*. It is worth noting that these genes often manifest as single gene mutations or multi-gene combined mutations during tumorigenesis and development, which presents a formidable challenge to current cancer treatment technologies based on single gene targets.

Gene therapy is an emerging and highly effective treatment method for complex diseases such as cancer induced by multiple gene mutations, for example, silencing activated oncogenes or supplementing tumor cells with normally functioning tumor suppressor genes to achieve functional cure. The effect of gene therapy is closely related to its delivery vector. The currently commonly used adeno-associated viruses (AAVs) and lentiviral vectors show great application potential in gene delivery. However, limited by the gene capacity of AAV vectors (~4.5kb) and lentiviral vectors (~8kb), they usually can only deliver 1-2 genes for treatment. In addition, the increase in the gene load of viral vectors may increase the structural instability of the virus and reduce the efficiency of multi-gene co-expression. More importantly, the safety issues of viral vectors themselves cannot be ignored.

### SUMMARY

The present disclosure aims to provide a multi-gene nanovector that has the potential to become a more efficient, safer and novel anti-cancer drug.

In order to achieve the above object, the first aspect of the present disclosure provides a multi-gene nanovector comprising a three-branch central ligand and gene combination sequences covalently connected on each branch of the three-branch central ligand, the gene combination sequences on each branch are the same;
the gene combination sequences comprise at least two tumor suppressor genes selected from TP53, BIM and PTEN successively in the direction extending outward from the three-branch central ligand.

The second aspect of the present disclosure provides a multi-gene nanovector comprising a three-branch central ligand and gene combination sequences covalently connected on each branch of the three-branch central ligand, the gene combination sequences on each branch are the same;
the gene combination sequences comprise tumor suppressor genes and a proto-oncogene successively in the direction extending outward from the three-branch central ligand, wherein the tumor suppressor genes is one selected from the group consisting of TP53, BIM and PTEN, and the proto-oncogene is MYC.

The third aspect of the present disclosure provides a method for preparing the multi-gene nanovector according to the aforementioned first aspect and/or the aforementioned second aspect, wherein the method comprises the following steps:
(1) Performing a Click cross-linking reaction on the three-branch central compound represented by formula (I) and a single-stranded primer nucleic acid with an azide modification at the 5' end to obtain a tri-branched primer containing 3 primers;
(2) Carrying out a branch PCR reaction in a PCR reaction system by using the tri-branched primer as a primer and using a linear DNA as a template to obtain the multi-gene nanovector; wherein the sequence information of the linear DNA is consistent with the gene combination sequences in the multi-gene nanovector according to the aforementioned first aspect and/or the aforementioned second aspect;

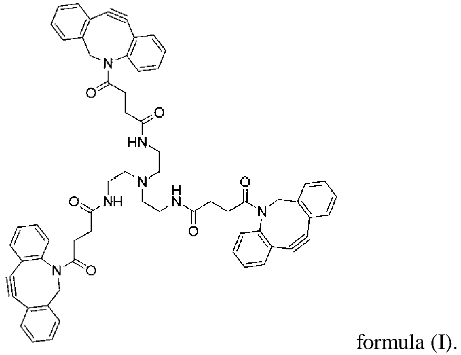

The fourth aspect of the present disclosure provides a use of a multi-gene nanovector according to the aforementioned first aspect and/or the aforementioned second aspect in the preparation of an anti-cancer drug.

The fifth aspect of the present disclosure provides an anti-cancer drug, wherein the anti-cancer drug consists of an active ingredient and an adjuvant, the active ingredient comprises the multi-gene nanovector according to the aforementioned first aspect and/or the aforementioned second aspect.

The technical solution provided by the present disclosure has at least the following advantages:
(1) The multi-gene nanovector provided by the present disclosure integrates various emerging gene regulation tools at different levels into a comprehensive tool kit and loads it into a chromatin-like payload. Guided by the central dogma, the multi-gene nanovector simulates the chromosome-mediated gene decoding process at different levels and multiple spatiotemporal dimensions, and realizes chromosome-mediated multi-level and multi-dimensional gene network regulation, thereby achieving the purpose of disease treatment. The strategy of simulating chromatin DNA to accurately regulate gene expression is expected to combat complex diseases in a manner close to chromosome regulation.
(2) The multi-gene nanovector provided by the present disclosure simultaneously loads the expression frames regulated by multiple genes into one entirety, and maintains the dimensions of cellular delivery, thereby achieving the balance in gene capacity, nanovector dimension, multi-gene co-expression, and safety of the multi-gene delivery nanovector .
(3) The technical solution provided by the present disclosure uses branch PCR technology to construct a DNA multi-gene nanovector comprising multi-component gene units (therapeutic gene targets such as tumor suppressor genes and proto-oncogenes) with different gene regulatory functions (overexpression, RNA interference and other gene regulatory technologies), and verifies the multi-gene co-expression or regulation ability, cell apoptosis induction ability and cell proliferation inhibition ability of this type of nanovector in different cancer cell types. Finally, using mice with different cancer cell xenografts as a model, the therapeutic efficacy and safety of this type of nanovector in xenograft tumors are further verified, thereby verifying that this type of multi-gene loaded multi-gene nanovector can achieve directional regulation of cancer cell apoptosis by balancing the expression level differences of tumor suppressor genes and proto-oncogenes from the two aspects of *in vitro* activity and *in vivo* activity. The technical solution provided by the present disclosure is conducive to synchronously balancing and regulating the genes related to tumorigenesis and development, further reducing the potential side effects, toxicity and tolerance on the basis of improving anti-cancer activity, thereby providing technical support for the development of more efficient and safe pan-cancer genome therapeutic methods.

### BRIEF DESCRIPTION OF THE DRAWINGS

FIG. 1 shows a schematic diagram of the synthesis of tri-branched primer;
FIG. 2 shows a schematic diagram of the sequence structure of the NP-TP53-BIM-PTEN vector;
FIG. 3 shows a schematic diagram of the sequence structure of the NP-TP53-shMYC vector;
FIG. 4 shows 1% agarose gel electrophoresis analysis of the branched PCR self-assembly products;
FIG. 5 shows an AFM characterization of NP-TP53-BIM-PTEN;
FIG. 6 shows an AFM characterization of NP-TP53-shMYC;
FIG. 7 shows a DLS characterization of NP-TP53-BIM-PTEN;
FIG. 8 shows a DLS characterization of NP-TP53-shMYC;
FIG. 9 shows the serum stability analysis of NP-TP53-BIM-PTEN;
FIG. 10 shows the serum stability analysis of NP-TP53-shMYC;
FIG. 11 shows an expression analysis of three genes mRNA of NP-TP53-BIM-PTEN in NCI-H1299 cells;
FIG. 12 shows an expression analysis of three genes proteins of NP-TP53-BIM-PTEN in NCI-H1299 cells;
FIG. 13 shows an expression analysis of two gene mRNAs of NP-TP53-shMYC in MDA-MB-231 cells;
FIG. 14 shows an expression analysis of two gene proteins of NP-TP53-shMYC in MDA-MB-231 cells;
FIG. 15 illustrates the comparative analysis of the apoptosis-inducing activity of multi-gene nanovectors, linear DNA and plasmids on NCI-H1299 cells;
FIG. 16 illustrates the comparative analysis of the proliferation inhibitory activity of multi-gene nanovectors, linear DNA and plasmids on NCI-H1299 cells;
FIG. 17 shows the comparative analysis of the apoptosis-inducing activity of NP-TP53-shMYC, NP-TP53 and NP-shMYC on MDA-MB-231 cells;
FIG. 18 shows an evaluation of the in vivo anti-tumor therapeutic effect of multi-gene nanovector;
FIG. 19 shows an analysis of TP53, BIM and PTEN gene expression levels in tumor tissues;
FIG. 20 shows the quantitative analysis of apoptosis markers and cell proliferation marker Ki67 in tumor tissues;
FIG. 21 shows the evaluation of the in vivo anti-tumor therapeutic effect of NP-TP53-shMYC multi-gene nanovector;
FIG. 22 shows an analysis of TP53 and MYC gene expression levels in tumor tissues;
FIG. 23 illustrates an analysis of TP53 and MYC expression levels in tumor tissues;
FIG. 24 shows an observation of HE tissue staining sections of different organs of MDA-MB-231 transplanted tumor-bearing mice;
FIG. 25 shows detection of immune inflammatory factors in the blood of MDA-MB-231 transplanted tumor-bearing mice;
FIG. 26 illustrates shows an observation of HE tissue staining sections of different organs of NCI-H1299 transplanted tumor-bearing mice;
FIG. 27 shows detection of immune inflammatory factors in the blood of NCI-H1299 transplanted tumor-bearing mice;
FIG. 28 illustrates an expression analysis of three genes mRNA of NP-PTB in NCI-H1299 cells;
FIG. 29 illustrate an expression analysis of three genes mRNA of NP-TBP-V in NCI-H1299 cells.

### DETAILED DESCRIPTION

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

In the present disclosure, the "selected...successively" in the technical feature "the gene combination sequences comprise at least two tumor suppressor genes selected from TP53, BIM and PTEN successively in the direction extending outward from the three-branch central ligand" refers to selecting two or three tumor suppressor genes from "TP53, BIM, PTEN" without changing the sequence front and back. Exemplarily, the gene combination sequences are TP53-BIM-PTEN, TP53-BIM, TP53-PTEN, and BIM-PTEN in the direction extending outward from the three-branch central ligand.

In the present disclosure, TP53, BIM and PTEN are all names of tumor suppressor genes, and MYC is the name of a proto-oncogene. p53 is the name of a protein expressed by TP53, c-Myc is the name of a protein expressed by MYC; BIM protein and PTEN protein are proteins expressed by BIM and PTEN, respectively.

In the present disclosure, PCR refers to polymerase chain reaction.

### Part 1

As previously mentioned, the first aspect of the present disclosure provides a multi-gene nanovector comprising a three-branch central ligand and gene combination sequences covalently connected on each branch of the three-branch central ligand, the gene combination sequences on each branch are the same;
the gene combination sequences comprise at least two tumor suppressor genes selected from TP53, BIM and PTEN successively in the direction extending outward from the three-branch central ligand.

Preferably, the terminals of the gene combination sequences further comprise a proto-oncogene MYC in the direction extending outward from the three-branch central ligand.

The inventors of the present disclosure have discovered in their research that in the multi-gene nanovector, the arrangement order of various tumor suppressor genes and proto-oncogenes in the direction extending outward from the three-branched central ligand has an important influence on overexpression and anti-cancer activity of the multi-gene nanovector. Under the arrangement order specifically required by the present disclosure, the overexpression condition, anti-cancer activity and biosafety of the prepared multi-gene nanovector are all excellent.

Preferably, the gene combination sequences comprise three tumor suppressor genes TP53, BIM and PTEN successively in the direction extending outward from the three-branch central ligand. The inventors of the present disclosure have discovered in their research that under the preferred condition, the multi-gene nanovector provided by the present disclosure has better anti-cancer activity and higher biosafety. In addition, the inventors of the present disclosure have also found in their research that TP53, BIM, and PTEN expression elements may require different types of promoters and transcription terminators to ensure the co-expression ability of the three genes.

In a case of preferably, the three-branch central ligand is provided by a three-branch central compound represented by formula (I);

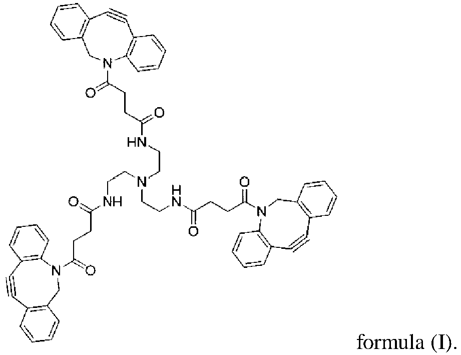

The present disclosure has no particular limitation on the specific method for preparing the three-branch central compound represented by formula (I), those skilled in the art can make a selection according to the technical means known in the art. However, in order to obtain the target compound with a higher yield, the present disclosure provides a preferred specific embodiment hereinafter, which will not be repeatedly described herein, and those skilled in the art should not interpret it as a limitation on the present disclosure.

### Part 2

As previously mentioned, the second aspect of the present disclosure provides a multi-gene nanovector comprising a three-branch central ligand and gene combination sequences covalently connected on each branch of the three-branch central ligand, the gene combination sequences on each branch are the same;

The gene combination sequences comprise tumor suppressor genes and a proto-oncogene successively in the direction extending outward from the three-branch central ligand, wherein the tumor suppressor genes is one selected from the group consisting of TP53, BIM and PTEN, and the proto-oncogene is MYC.

Preferably, the gene combination sequences comprise TP53 and MYC successively in the direction extending outward from the three-branch central ligand. The inventors of the present disclosure have discovered in their research that under the preferred condition, the multi-gene nanovector provided by the present disclosure has better anti-cancer activity and higher biosafety.

In a case of preferably, the three-branch central ligand is provided by a three-branch central compound represented by formula (I).

The present disclosure has no particular limitation on the specific method for preparing the three-branch central compound represented by formula (I), those skilled in the art can make a selection according to the technical means known in the art. However, in order to obtain the target compound with a higher yield, the present disclosure provides a preferred specific embodiment hereinafter, which will not be repeatedly described herein, and those skilled in the art should not interpret it as a limitation on the present disclosure.

### Part 3

As previously mentioned, the third aspect of the present disclosure provides a method for preparing the multi-gene nanovector according to the aforementioned first aspect and/or the aforementioned second aspect, wherein the method comprises the following steps:
(1) performing a Click cross-linking reaction on the three-branch central compound represented by formula (I) and a single-stranded primer nucleic acid with an azide modification at the 5' end to obtain a tri-branched primer containing 3 primers;
(2) Carrying out a branch PCR reaction in a PCR reaction system by using the tri-branched primer as a primer and using a linear DNA as a template to obtain the multi-gene nanovector; wherein the sequence information of the linear DNA is consistent with the gene combination sequences in the multi-gene nanovector according to the aforementioned first aspect and/or the aforementioned second aspect;

The term "in a PCR reaction system" mentioned in the present disclosure means that under the condition of having the necessary materials required for the PCR reaction, in addition to using the tri-branched primer as a primer and using a linear DNA as a template, the multi-gene nanovector further comprises necessary materials such as nucleotides, DNA polymerase and buffer solution, so that the branch PCR reaction can be successfully carried out.

The present disclosure has no particular limitation on the programmed reaction conditions for conducting the branch PCR reaction, and those skilled in the art can make routine designs based on technical means known in the art. The present disclosure exemplifies a preferred specific embodiment hereinafter, which should not be construed as a limitation on the present disclosure by those skilled in the art.

The preparation method of the multi-gene nanovector according to the present disclosure further comprises post-processing means known in the art such as purification and recovery, which will not be repeatedly described herein and should not be construed as a limitation on the present disclosure by those skilled in the art.

### Part 4

As previously mentioned, the fourth aspect of the present disclosure provides a use of the multi-gene nanovector according to the aforementioned first aspect and/or the aforementioned second aspect in the preparation of an anti-cancer drug.

Preferably, the anti-cancer drug is at least one selected from the group consisting of an anti-breast cancer drug, an anti-lung cancer drug, an anti-liver cancer drug, an anti-colorectal cancer drug, and an anti-prostate cancer drug.

More preferably, the anti-cancer drug is an anti-breast cancer drug and/or an anti-lung cancer drug.

Preferably, the anti-cancer drug is administered by orthotopic intratumoral injection and/or intravenous injection.

### Part 5

As previously mentioned, the fifth aspect of the present disclosure provides an anti-cancer drug, wherein the anti-cancer drug consists of an active ingredient and an adjuvant, wherein the active ingredient comprises the multi-gene nanovector according to the aforementioned first aspect and/or the aforementioned second aspect.

Preferably, content of the active ingredient is within the range of 0.01-99.99wt%, more preferably within the range of 0.1-99.9wt%, such as 0.01 wt%, 0.1 wt%, 1 wt%, 3 wt%, 5 wt%, 10 wt%, 15 wt%, 20 wt%, 25 wt%, 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, 95 wt%, 99 wt%.

In a case of preferably, the adjuvant comprises liposome 2000 and/or liposome 3000. The inventors of the present disclosure have found in their research that when the anti-cancer drugs comprise liposome 2000 and/or liposome 3000 as the adjuvant, the active ingredient in said multi-gene nanovector may have a more efficient anticancer activity.

The present disclosure will be described in detail below with reference to examples. In the following examples, unless otherwise specified, all the raw materials are commercially available. Unless otherwise specified in the present disclosure, the room temperature refers to 25 ±2 °C.

### Preparation Example 1: preparation of tri-branched primer

### 1) Synthesis of a three-branch central compound represented by formula (I)

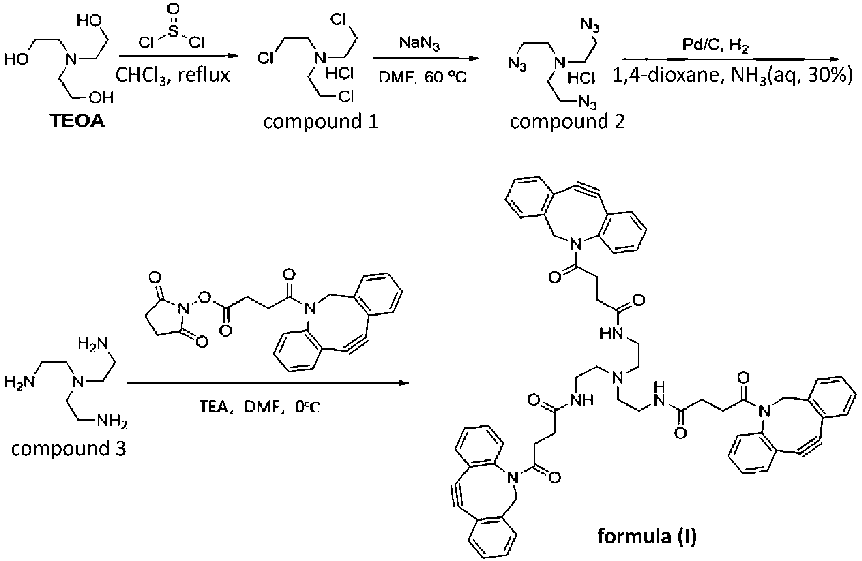

Synthesis steps of compound 1: 10.0mmol of triethanolamine (TEOA) was dissolved in 50mL of anhydrous chloroform, 135mmol of thionyl chloride was dropwise added, and reflux was performed for 3h. The reduced pressure distillation was carried out to remove the solvent, 50mL of dichloromethane was added, and filtered to obtain 2.21g of white needle-shaped solid with a yield of 92%. ¹H NMR (400 MHz, d*₆*-acetone): δ (ppm) 4.23 (t, *J* = 7.2 Hz, 6H), 3.73 (t, *J* = 7.2 Hz, 6H). ¹³C NMR (100 MHz, *d*₆-acetone): δ (ppm) 56.01, 38.57.

Synthesis steps of compound 2: 5.00mmol of compound 1 was dissolved in 25mL of N,N-dimethylformamide (DMF), 30.0mmol of sodium azide was added, and stir and reaction were performed at 60 °C for 3h. 50mL of ethyl acetate was added, and the reaction product was washed with saturated sodium bicarbonate, water, and saturated saline solution for 8 times, dried with anhydrous sodium sulfate, filtered and concentrated to obtain an oily substance, and 4M HCl ethyl acetate solution was added to obtain 1.08g of a white needle-shaped solid with a yield of 83%. ¹H NMR (400 MHz, CDCl₃): δ (ppm) 4.06 (t, *J* = 5.2 Hz, 6H), 3.41 (t, *J* = 5.2 Hz, 6H). ¹³C NMR (100 MHz, CDCl₃): δ (ppm) 52.89, 45.84.

The synthesis steps of compound 3 were as follows: 4.15mmol of compound 2 was dissolved in 15mL of 1,4-dioxane, 44.2mg of palladium carbon (10wt%) was added, and 5mL of ammonia water (30wt%) was added, vacuumization was performed and H₂ was then introduced, stir and reaction were performed at room temperature for 3h. After the reaction was completed, filtration with diatomaceous earth was performed. Ethyl acetate (3×10mL) and water were subsequently added for extraction, and then washed with saturated NaHCO₃ solution. After separation, the organic phase was dried with anhydrous MgSO₄ and filtered, and the filtrate was evaporated to dryness and drained to obtain a yellow oily product, which was directly subjected to the next step.

The synthesis steps of the compound represented by formula (I): under argon protection, the compound 3 (0.42mmol) was dissolved with 10mL of dry DMF in two round-bottom flasks having a volume of 50mL respectively, diphenylcyclooctyne-active ester (DBCO-NHS Ester, 1.38mmol) was subsequently added, and cooled to 0 °C, and triethylamine (TEA, 1.26mmol) was added in an ice-water bath (0 °C), reacted in an ice-water bath (0 °C) for 1h, and then heated to room temperature to react overnight. After the reaction was complete by spot plate detection, 10mL of water and ethyl acetate (4×10 mL) were added to the flask for extraction, and all organic phases were combined after separation, and an appropriate amount of anhydrous Na₂SO₄ was added for drying, and then filtered, and the filtrate was subjected to reduced pressure distillation by a rotary evaporator to remove the solvent, and then directly subjected to column chromatography separation (dichloromethane/methanol=30:1, v/v), 296.1mg of a light yellow viscous product compound was obtained with a yield of 70%. ¹H NMR (400 MHz, CDCl₃); δ (ppm) 7.64 (m, 12H): 7.35 (m, 12H), 3.34 (s, 6H), 2.76 (s, 12H), 2.60 (s, 6H), 2.51 (s, 6H). ¹³C NMR (100 MHz, CDCl₃): δ (ppm) 172.52, 151.12, 148.26, 131.96, 129.50, 128.69, 128.15, 127.92, 127.60, 126.95, 125.44, 123.20, 123.08, 122.36, 114.51, 107.77, 77.47, 77.36, 77.16, 76.84, 55.57, 53.50, 45.86, 29.79, 29.64, 8.65. HRMS: calculated for [M+Na]⁺ 1030.4268, found 1030.4265.

### 2) Preparation of tri-branched primers

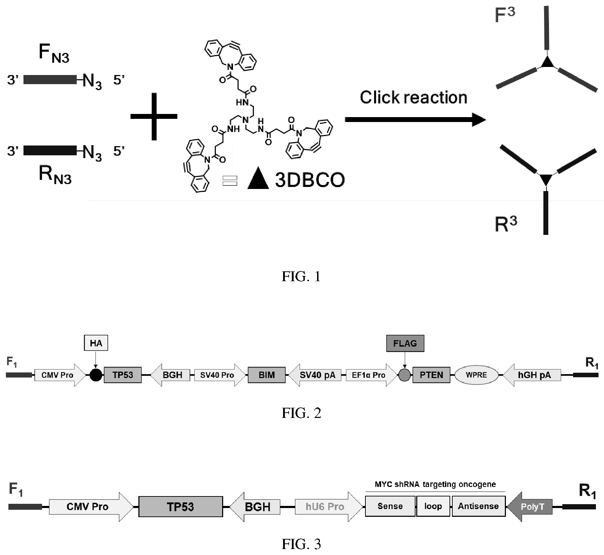

After the mass spectrometry characterization was correct, the cyclooctalkynyl group on the three-branch central compound represented by formula (I) was used to perform a Click cross-linking reaction with an oligodeoxyribonucleic acid molecule having an azide modification at the 5' end, such that a tri-branched primer containing three primers was obtained. The schematic diagram of the synthesis of tri-branched primer was shown in FIG. 1. The sequence information of the tri-branched primers was shown in Table 1.

**Table 1. Sequence information of tri-branched primers**

| Primer name | Sequence (5'-3') |
|---|---|
| F_{N3} (sequence 1) | CCTCCCAAGATGGCCCGATA |
| R_{N3} (sequence 2) | CCTCCTCTTCCCTGTCCAAA |

The specific synthesis process of tri-branched primers was as follows: the primers F_{N3} and R_{N3} were custom-made (by Sangon Biotech (Shanghai) Co., Ltd.) according to the primer sequence in Table 1. First, the single-stranded nucleic acid (10OD) with an azide modification at the 5' end was dissolved in 200µL of double distilled water (ddH₂O), and the small molecule three-branch central compound dissolved in DMF (nucleic acid: three-branch central compound = 3.3:1) was then added into the centrifuge tube, the materials were vibrated and reacted overnight (10 hours) in a metal bath at the temperature of 37 °C in a PBS buffer solution with a final concentration of 20mM. After completion of the reaction, the gel was cut and purified by using 8% denaturing polyacrylamide gel (PAGE gel).

The purification process of the tri-branched primer was as follows: the target band was cut under ultraviolet light, and cut into small particles and transferred to a 15mL centrifuge tube, 0.3M of NaOAc extraction solution was then added into the centrifuge tube until the liquid volume was about twice that of the colloid particles, and rotated overnight for extraction. After the extraction was completed, centrifugation was performed, the supernatant was collected and transferred to a new 15mL centrifuge tube, the pre-cooled anhydrous ethanol at a temperature of -20 °C with a volume of three times of the supernatant was added into the centrifuge tube loaded with the supernatant, the centrifuge tube was turned upside down and mixed uniformly, then placed in a refrigerator with a temperature of -20 °C to carry out the low-temperature precipitation for 2 hours. After completion of the precipitation, the centrifuge tube was subjected to centrifugation at the temperature of 4 °C for 30 minutes, the supernatant was poured out after completion of the centrifugation. 2mL of 75% ethanol that has been pre-cooled at -20 °C was then added into the centrifuge tube with the precipitate. Centrifugation was subsequently performed at the temperature of 4 °C for 20 minutes, the supernatant was poured out after completion of the centrifugation, the vacuum dry was then carried out to remove the residual ethanol in the centrifuge tube. After completion of the drying process, 200 µL of ddH₂O was added to the centrifuge tube, which was rotated to dissolve the precipitate, the concentration was measured, and the centrifuge tube was stored in a refrigerator with the temperature of -20 °C.

The purified tri-branched primers were characterized and analyzed by HRMS mass spectrometry. As shown in Table 2, the difference between the mass spectrometry results of the triplets F³ and R³ with the theoretical values were within the allowable error range, indicated that the two tri-branched primers were synthesized correctly and can be used for subsequent branch PCR reaction to construct the multi-gene nanovector.

**Table 2 Mass spectrometry characterization of branch primers**

| Triplet primers | F³ | R³ |
|---|---|---|
| MW (Calcd) | 20019.8 | 19650.6 |
| MW (Found) | 20020.6 | 19651.8 |

### Preparation Example 2: vector design and construction of linear DNA template L-TP53-BIM-PTEN

The synchronous expression strategy of multi-component tumor suppressor genes adopted the multi-gene nanovector elements that integrated the overexpression of three tumor suppressor genes (TP53, PTEN, and BIM), named NP-TP53-BIM-PTEN, and its linear DNA template was named L-TP53-BIM-PTEN. The sequence information of L-TP53-BIM-PTEN included the open reading frames of the three genes TP53, BIM, and PTEN and their respective promoters and terminators, which in order from the 5' end to the 3' end were: F₁ box, CMV promoter, HA tag, TP53 ORF, BGH terminator, SV40 promoter, BIM ORF, SV40 pA terminator, EF1α promoter, FLAG tag, PTEN ORF, WPRE, hGH pA terminator and R₁ box element, with a sequence length of 7,000bp. The schematic diagram of the sequence structure of the NP-TP53-BIM-PTEN vector was shown in FIG. 2.

The construction template of L-TP53-BIM-PTEN was derived from the plasmid template P-TP53-BIM-PTEN. The construction of the plasmid template adopted the Golden gate assembly attachment method. The specific process was as follows:

### 1) PCR amplification and gel recovery to obtain linearized vector and insertion fragment

According to Table 3, different plasmids were used as templates and different primers were used to amplify DNA fragments of different lengths by means of the PCR method. The PCR reaction system was composed of 50 ng of plasmid template, 2.5 µM of upstream and downstream primers, and 1×Q5 Hot Start High-Fidelity Master Mix (New England Biolabs). The PCR reaction program was as follows: 98 °C, 30s - (98 °C, 10s - 60 °C, 10s - 72 °C, 1min) × 30cycles - 72 °C, 2min. The names of the obtained PCR products were L-HA-TP53-BGH, L-SV40, L-BIM, L-SV40 polyA, L-EF1a, L-fPTEN, and L-WPRE-hGHpA respectively. These PCR products were purified by gel recovery kit Gel DNA recovery kit (ZYMO) and used for subsequent gene assembly reactions.

**Table 3 Primer sequences for construction of P-TP53-BIM-PTEN**

| Plasmid template | Primer name | Primer sequence (5'-3') * |
|---|---|---|
| P-TP53 | TBfPV2-F1 (sequence 3) | GTCACACCTGCTAATTCGCGGCGGGTGTGGT GGTTACGCGCAGCGTGA |
| | TBfPV2-R1 (sequence 4) | GTCACACCTGCTAATGCTTAATGCGCCGCTA CAGGGCGCGTGGGGA |
| pGL4.13 | TBfPV2-F2 (sequence 5) | GTCACACCTGCTAATAAGCGCGCAGCAC CATGGCCTGAAATAA |
| | TBfPV2-R2 (sequence 6) | GTCACACCTGCTAATATGGTGGCTTTACCAA CAGTACCGGA |
| P-BIM | TBfPV2-F3 (sequence 7) | GTCACACCTGCTAATCCATGGCAAAGCAAC CTTCTGATGTAA |
| | TBfPV2-R3 (sequence 8) | GTCACACCTGCTAATTATCAATGCATTCTCC ACACCAGGCGGA |
| pGL4.13 | TBfPV2-F4 (sequence 9) | GTCACACCTGCTAATGATAATTCTAGAGTCG GGGCGGCCGGCCGCTTCGA |
| | TBfPV2-R4 (sequence 10) | GTCACACCTGCTAATAGCCTACCACATTTGT AGAGGTTTTA |
| pXR001 (Addgene number: 109049) | TBfPV2-F5 (sequence 11) | GTCACACCTGCTAATGGCTCCGGTGCCCGTC AGTGGGCAGA |
| | TBfPV2-R5 (sequence 12) | GTCACACCTGCTAATCCGTACGTCACGACAC CTGAAATGGAAGA |
| P-PTEN | TBfPV2-F6 (sequence 13) | GTCACACCTGCTAATACGGCCACCATGGACT ACAAAGACCATGACGGTGA |
| | TBfPV2-R6 (sequence 14) | GTCACACCTGCTAATATTCTCAGACTTTTGT AATTTGTGTATGCTGA |
| AAV: ITR-U6-sgRNA(backbone)-p CBh-Cre-WPRE-hGHpA-IT R (Addgene number: 60229) | TBfPV2-F7 (sequence 15) | GTCACACCTGCTAATGAATTCGATATCAAGC TTATCGATAA |
| | TBfPV2-R7 (sequence 16) | GTCACACCTGCTAATGCGAAAGGACAGGGA AGGGAGCAGTGGTTCA |

| | | |
|---|---|---|
| Note: * bold font denoted the PaqCI recognition sites. | | |

### 2) Construction of plasmid template P-TP53-BIM-PTEN with the Golden Gate ligation method

According to Table 4, the purified DNA fragments of different lengths were prepared, and subjected to the assembly and reaction according to the following temperature program, the specific program was (37 °C, 5min - 16 °C, 5min) × 30 cycles - 37 °C, 5min, 60 °C, 5min, 16 °C, 30min. After the reaction was completed, 50µL of STabl4 Chemically Competent Cell (ZC1015) competent cells were taken out from -80°C and placed in an ice bath to thaw, 5µL of the ligation product was further added into the 50µL of competent cells, and the mixture was quickly and gently blown evenly, placed in an ice bath for 30min, and then heat-shocked at a temperature of 42 °C for 90s, and the centrifuge tube was quickly transferred to an ice bath to cool the cells for 2-3min, and 100µL of sterile LB culture medium (free from antibiotics) was finally added, after the materials were mixed uniformly, the mixture was directly added onto the Luria-Bertani (LB) solid medium containing the corresponding antibiotic ampicillin, the solid medium was evenly spread with sterile plate-coated glass beads, then inverted and cultured at the temperature of 37 °C for 16h. After the bacterial colonies were formed, a single colony was selected and placed in 10 mL of sterile LB liquid culture medium, 10 µL of ampicillin (the final concentration was 100 µg/mL) was added, and cultured at 37°C for 16 hours. The bacterial solution was sent to Beijing Tsingke Biotechnology Co., Ltd. for sequencing analysis.

**Table 4 Golden Gate Assembly ligation reaction System**

| Component (fragment length) | Volume (amount of substance) |
|---|---|
| 10×T4 ligation | 2 µL |
| L-HA-TP53-BGH (5500 bp) | 4 µL (22 fmol) |
| L-SV40 (448 bp) | 0.2 µL (55 fmol) |
| L-BIM-ORF (323 bp) | 0.2 µL (55 fmol) |
| L-SV40 polyA (254 bp) | 0.6 µL (55 fmol) |
| L-EF1a (1178 bp) | 0.5 µL (55 fmol) |
| L-fPTEN-ORF (1307 bp) | 0.7 µL (55 fmol) |
| L-WPRE-hGHpA (1117 bp) | 0.5 µL (55 fmol) |
| PaqCI Activator (20 µM) | 0.5 µL |
| PaqCI (10 U/µL) | 1 µL |
| T4 DNA ligase (2000 U/µL) | 0.5 µL |
| ddH₂O | 9.3 µL |
| Total | 20 µL |

### 3) Amplification and recovery of the linear DNA template L-TP53-BIM-PTEN

After sequencing verification result was correct, the plasmid P-TP53-BIM-PTEN was amplified, cultured and extracted. The specific process was as follows: 1 mL of glycerol tube was added to 50 mL of LB liquid culture medium, shaken with a rotational speed of 200 rpm by a shaking table at 37 °C for 16-18 hours, and the plasmid was extracted using a high-purity endotoxin-free plasmid large-scale plasmid kit (CoWin Bioscience Co., Ltd.), and the concentration was then measured by using NanoDrop 2000. The plasmid P-TP53-BIM-PTEN was used as a template, PCR amplification was performed according to the primers in Table 5 (L-V2-F and L-V2-R). The amplification reaction involved with 50 ng of plasmid template, 2.5 µM upstream and downstream primers and 1×Q5 Hot Start High-Fidelity Master Mix (New England Biolabs). The PCR reaction program was 98 °C, 30 s - (98 °C, 10 s - 60 °C, 10 s - 72 °C, 1 min) × 30 cycles - 72 °C, 2 min. The PCR products were separated by 1% agarose gel electrophoresis, the gel was recovered and purified by using a gel purification kit (QIAGEN).

**Table 5 Primers for linear DNA template amplification**

| Primer name | Sequences (5'-3') |
|---|---|
| L-V2-F (sequence 17) | CCTCCCAAGATGGCCCGATACGATGTACGGGCCAGATATACG CGTTGA |
| L-V2-R (sequence 18) | CCTCCTCTTCCCTGTCCAAAAAGGACAGGGAAGGGAGCAGTG GTTCA |

### Preparation Example 3: Design and construction of vector of linear DNA template TP53-shMYC

The bidirectional regulation of tumor suppressor gene overexpression in combination with downregulation of proto-oncogene was performed by using an element integrating the overexpression of tumor suppressor genes TP53 and the shRNA expression of down-regulated MYC, the element was named NP-TP53-shMYC, and its linear DNA template was named L-TP53-shMYC. The sequence information of L-TP53-shMYC included the promoters and terminators of TP53 and MYC shRNA respectively, from the 5' end to the 3' end in sequence: F₁ box, CMV promoter, TP53 ORF, BGH terminator, hU6 promoter, MYC shRNA and poly-T terminator and R₁ box element, with a sequence length of 2,932bp. The schematic diagram of the sequence structure of the NP-TP53-shMYC vector was shown in FIG. 3.

The construction template of L-TP53-shMYC was derived from the plasmid template P-TP53-shMYC. The construction of the plasmid template adopted the seamless cloning assembly connection method. The specific process was as follows:

### 1) PCR amplification and gel recovery to obtain linearized vector and insertion fragment

The DNA fragments with different lengths were amplified by using different plasmids as templates and using different primers with the PCR method according to Table 6. The PCR reaction system included 50 ng plasmid template, 2.5 µM upstream and downstream primers and 1×Q5 Hot Start High-Fidelity Master Mix (New England Biolabs). The PCR reaction program was 98 °C, 30s - (98 °C, 10s - 60 °C, 10s - 72 °C, 1min) ×30 cycles - 72 °C, 2min. The obtained PCR products were named L-TP53 and L-shMYC respectively. These PCR products were purified by Gel DNA recovery kit (ZYMO) and used for subsequent gene assembly reactions.

**Table 6 Primer sequences for construction of P-TP53-shMYC**

| Plasmid template | Primer name | Primer sequence (5'-3') * |
|---|---|---|
| P-TP53 | TP53-F1 (sequence 19) | GGCGGGTGTGGTGGTTACGCGCAGC |
| | TP53-R1 (sequence 20) | GCGCTTAATGCGCCGCTACAGGGCG |
| shMYC | MYC-F2 (sequence 21) | CGCCCTGTAGCGGCGCATTAAGCGCGAGG GCCTATTTCCCATGAT |
| | MYC-R2 (sequence 22) | GCTGCGCGTAACCACCACACCCGCCCTCGA GAAAAAAGGAAACGA |

### 2) Construction of plasmid template P-TP53-shMYC by seamless cloning assembly ligation method

According to Table 7, the purified DNA fragments of different lengths were prepared, and subjected to the assembly and reaction according to the following temperature program, the specific program was as follows: 37 °C, 15min - 16 °C, 30min. After the reaction was completed, 50µL of STabl4 Chemically Competent Cell (ZC1015) competent cells were taken out from -80 °C and placed in an ice bath to thaw, 5µL of the ligation product was further added into the 50µL of competent cells, and the mixture was quickly and gently blown evenly, placed in an ice bath for 30min, and then heat-shocked at a temperature of 42 °C for 90s, and the centrifuge tube was quickly transferred to an ice bath to cool the cells for 2-3min, and 100µL of sterile LB culture medium (free from antibiotics) was finally added, after the materials were mixed uniformly, the mixture was directly added onto the Luria-Bertani (LB) solid medium containing the corresponding antibiotic ampicillin, the solid medium was evenly spread with sterile plate-coated glass beads, then inverted and cultured at the temperature of 37 °C for 16h. After the bacterial colonies were formed, a single colony was selected and placed in 10 mL of sterile LB liquid culture medium, 10 µL of ampicillin (the final concentration was 100 µg/mL) was added, and cultured at 37 °C for 16 hours. The bacterial solution was sent to Beijing Tsingke Biotechnology Co., Ltd. for sequencing analysis.

**Table 7. Seamless clonal assembly ligation reaction system**

| Component (fragment length) | Volume (amount of substance) |
|---|---|
| 5×SE Cloning buffer | 2 µL |
| L-TP53 | 1 µL(22 fmol) |
| L-shMYC | 1 µL(55 fmol) |
| ddH₂O | 6 µL |
| Total | 10 µL |

### 3) Amplification and recovery of the linear DNA template L-TP53-shMYC

After sequencing verification result was correct, the plasmid L-TP53-shMYC was amplified, cultured and extracted. The specific process was as follows: 1 mL of glycerol tube was added to 50 mL of LB liquid culture medium, shaken with a rotational speed of 200 rpm by a shaking table at 37 °C for 16-18 hours, and the plasmid was extracted using a high-purity endotoxin-free plasmid large-scale plasmid kit (CoWin Bioscience Co., Ltd.), and the concentration was then measured by using NanoDrop 2000 (ultra-micro spectrophotometer). The plasmid L-TP53-shMYC was used as a template, PCR amplification was performed according to the primers in Table 8 (L-TM-F and L-TM-R). The amplification reaction involved with 50 ng of plasmid template, 2.5 µM upstream and downstream primers and 1×Q5 Hot Start High-Fidelity Master Mix (New England Biolabs). The PCR reaction program was 98 °C, 30 s - (98 °C, 10 s - 60 °C, 10 s - 72 °C, 1 min) × 30 cycles - 72 °C, 2 min. The PCR products were separated by 1% agarose gel electrophoresis, the gel was recovered and purified by using a gel purification kit (QIAGEN).

**Table 8 Primers for linear DNA template amplification**

| Primer name | Sequences (5'-3') |
|---|---|
| L-TM-F (sequence 23) | CCTCCCAAGATGGCCCGATAAGCTACAACAAGGCAAGGCT |
| L-TM-R (sequence 24) | CCTCCTCTTCCCTGTCCAAACTCGAGAAAAAAGGAAACGA |

### Example 1: Construction of multi-gene nanovectors NP-TP53-BIM-PTEN and NP-TP53-shMYC

The branch PCR reaction system was prepared according to Table 9. The linear DNA templates were L-TP53-BIM-PTEN and L-TP53-shMYC respectively, and the primers were tri-branched primers F³ and R³, with a final concentration of 0.25 µM. The branched PCR reaction program was 95 °C, 3min - (95 °C, 30s - 58 °C, 30s - 72 °C, 4min) × 35 cycles - 72 °C, 5min - 4 °C, 8h. The PCR product was recovered by using a PCR purification kit (Thermo Fisher), and the concentration was measured after elution, and the purity of the product was analyzed by using 1% agarose gel electrophoresis.

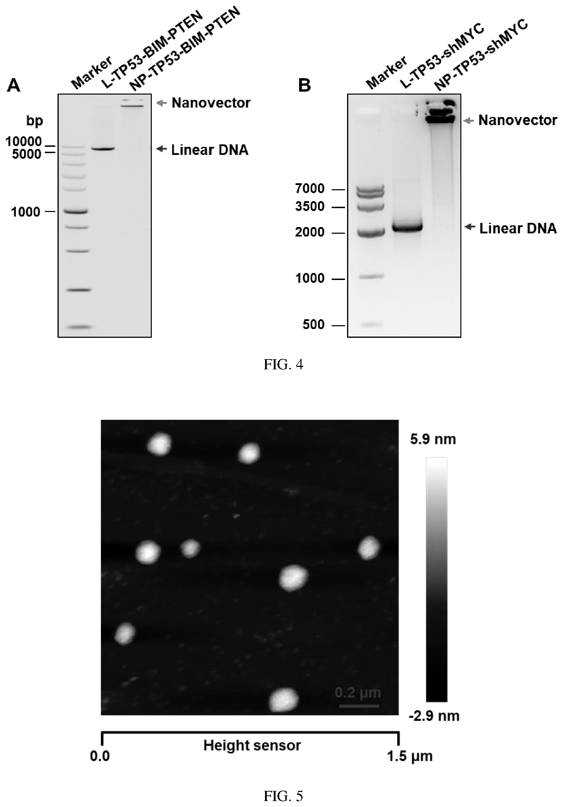

The results are shown in FIG. 4, wherein A represents NP-TP53-BIM-PTEN and B represents NP-TP53-shMYC. As can be seen, all the NP-TP53-BIM-PTEN and NP-TP53-shMYC are located in the gel wells and cannot move down, and there are no band at the places corresponding to the linear DNA templates L-TP53-BIM-PTEN and L-TP53-shMYC, indicating that the multi-gene nanovector constructed with the branch PCR is successfully synthesized.

**Table 9 The branch PCR reaction systems**

| Components | Volume (µL) |
|---|---|
| Linear DNA template (20 ng/µL) | 1 |
| F³ (with a final concentration of 0.25 µM) | 1 |
| R³ (with a final concentration of 0.25 µM) | 1 |
| ddH₂O | 22 |
| 2×Es Taq MasterMix | 25 |

### Test Example 1: Characterization of the physicochemical properties of multi-gene nanovectors NP-TP53-BIM-PTEN and NP-TP53-shMYC

### 1) Atomic Force Microscopy (AFM) characterization

5 µL of multi-gene nanovector with a concentration of 20 ng/µL was taken and dropwise added onto a new clean mica sheet, which was placed in a clean box and waited for drying naturally, and scanning observation was performed under AFM tap mode.

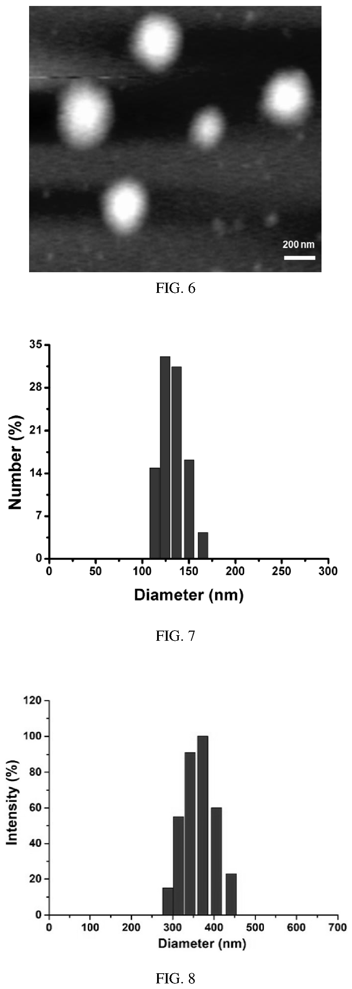

As can be seen from the results shown in FIG. 5 and FIG. 6, the AFM results indicated that the average particle size of the branch PCR product NP-TP53-BIM-PTEN nanoparticles was 104.8±24.2nm, and the average particle size of NP-TP53-shMYC was 350±30nm.

### 2) Dynamic Light Scattering (DLS) characterization

After dust removal through a 0.22 µm filter membrane, 80 µL of multi-gene nanovectors with a concentration about 200 ng/µL were added into a micro-cuvette, and subjected to the DLS detection under the temperature condition of 25 °C.

As can be seen from the results shown in FIG. 7 and FIG. 8, the DLS results indicated that in the aqueous solution state, the hydrodynamic diameter of NP-TP53-BIM-PTEN was 139.1±25.8nm, and the hydrodynamic diameter of NP-TP53-shMYC was 300-450nm.

### 3) Serum stability characterization

The stabilities of multigene nanovectors, linear DNA, and plasmids were compared in 30% fetal bovine serum (FBS) and observed and analyzed by using the agarose gel electrophoresis.

Linear DNA incubation system: 2 µg of linear DNA, 6 µL of 100% FBS, 2 µL of 100 mM PBS, supplemented with ddH2O to 20 µL (8 copies), incubated at 37 °C for 0h, 2h, 4h, 8h, 12h, 24h, 48h and 72h, respectively.

Plasmid incubation system: 2 µg of plasmid, 6 µL of 100% FBS, 2 µL of 100 mM PBS, supplemented with ddH2O to 20 µL (8 copies), incubated at 37°C for 0h, 2h, 4h, 8h, 12h, 24h, 48h and 72h, respectively.

Multi-gene nanovector incubation system: 2 µg of multi-gene nanovector, 6 µL of 100% FBS, 2 µL of 100 mM PBS, supplemented with ddH2O to 20 µL (8 copies), incubated at 37°C for 0h, 2h, 4h, 8h, 12h, 24h, 48h and 72h, respectively.

At each independent time point, 2.2 µL of 1% SDS was added, and the incubation was continuously performed at 37 °C for 5 min. The cells were then centrifuged at 13,000 rpm and a condition of 4 °C for 15 min. The supernatant was extracted and then added with 6 × DNA loading buffer and analyzed by agarose gel electrophoresis.

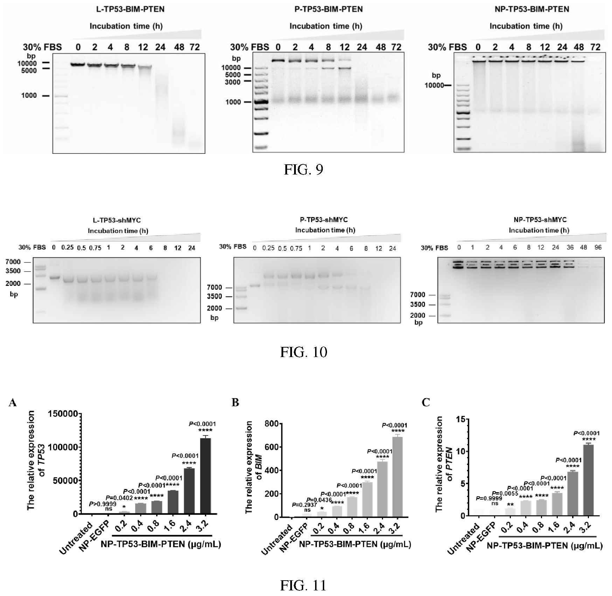

The results are shown in FIG. 9 and FIG. 10. As illustrated by FIG. 9, compared with linear DNA and plasmids, the multi-gene nanovector has higher serum stability, for example, L-TP53-BIM-PTEN and P-TP53-BIM-PTEN are completely degraded at 24 hours, while NP-TP53-BIM-PTEN is completely degraded until 72 hours. As can be seen from FIG. 10, for NP-TP53-shMYC, the multi-gene nanovector has higher serum stability than the linear DNA and plasmids, for example, L-TP53-shMYC and P-TP53-shMYC are completely degraded at 2 hours, while NP-TP53-BIM-PTEN is completely degraded until 96 hours.

### Test Example 2: Multi-gene nanovector NP-TP53-BIM-PTEN simultaneously overexpresses three tumor suppressor genes in lung cancer cells

### 1) Cell transfection

The type of cell culture medium was directly related to the cell type. The present disclosure was exemplified by non-small cell lung cancer cells NCI-H1299 to illustrate the cell expression and activity analysis process of multi-gene nanovectors in NCI-1299 cells. The specific cell transfection process was as follows:
a) When the cells were passaged, 2 mL of the cell suspension was added into 10 mL of RPMI 1640 complete medium, mixed and then added to a 6-well plate with 2 mL/well of the cell suspension to ensure that the cells were evenly distributed and the cell abundance was about 60-70%. After culture at the temperature condition of 37 °C and 5% CO₂ for 12-16 hours, the multi-gene nanovector was transfected into NCI-H1299 cells by using the transfection reagent Lipofectamine 3000 (Lipofectamine 3000 kit, purchased from Invitrogen Corporation);
b) Dilution of Lipofectamine 3000 with Opti-MEM: 5 µL of Lipofectamine 3000 was diluted with 195 µL of serum-free medium Opti-MEM, mix gently with a pipette, and placed at room temperature for 5 minutes;
c) Dilution of the gene vector with Opti-MEM: the transfection concentration was calculated according to 1.6 µg/mL, 1.6 µg of multi-gene nanovector, 5 µL of P3000 auxiliary packaging components and Opti-MEM were taken, with the total volume of 200 µL, the materials were added into a 1.5 mL centrifuge tube, and mixed gently with a pipette. The same mode was applies to other transfection masses;
d) The Lipofectamine 3000 liposomes diluted with Opti-MEM and the multi-gene nanovectors diluted with Opti-MEM were mixed uniformly with an equal volume and placed at room temperature for 15 minutes;
e) The original RPMI 1640 complete medium in the 6-well plate was discarded, 600 µL of Opti-MEM medium was added, and 400 µL of the mixed solution in step d) was evenly and dropwise added into the corresponding wells, and the 6-well plate was gently shaken such that the mixed solution was evenly distributed. After the cells were returned to the cell culture incubator at 37 °C and 5v% CO₂ for 4 hours, 2mL/well RPMI 1640 complete medium was replaced, and continued to culture for 48 hours, the total RNA and total protein were extracted for subsequent fluorescence quantification and protein blotting analysis (western blot analysis).

### 2) Fluorescence quantitative PCR

48h after transfection, cells were collected, and total RNA was extracted using an animal tissue/cell RNA extraction kit (CoWin Bioscience Co., Ltd.). RNA was used as a template to synthesize the first strand of cDNA using an RNA reverse transcription kit (TransGen Biotech). The reverse transcription reaction system was: 1µg of RNA, 1µL of gDNA Remover, 4µL of 5×TransScript^{®} All-in-One SuperMix for qPCR, supplemented with nuclease-free ultrapure water (Nuclease-free H₂O) to 1µL. The reverse transcription reaction procedure was: 42°C, 15min - 85°C, 5s. Preparation of the fluorescent quantitative PCR reaction system: 1µL of cDNA, 0.5µL of quantitative primer (10µM), 10µL of 2×PerfectStart^{®} Green qPCR SuperMix (TransGen Biotech), supplemented with Nuclease-free H₂O to 20µL. The fluorescence quantitative PCR program was 94°C, 5min - (94°C, 5s - 58°C, 20s - 72°C, 5min) × 40 cycles. The relative expression quantity analysis of different genes was calculated using the 2-^{ΔΔCq} method.

### 3) Western blot analysis

48 hours after the cells were transfected, the cells were harvested and the supernatant was discarded. An appropriate volume of RIPA lysis buffer was added into the cell pellet, and about 100 µL of RIPA lysis buffer was required for every 10⁶ cells. The cell pellet was blown open several times with a micropipette, and then placed on ice for lysis for 0.5 hour. After the cells were fully lysed, a centrifuge tube with a volume of 1.5 mL was placed in a 4 °C centrifuge and centrifuged at a rotational speed of 12,000 rpm for 10 minutes. The supernatant was transferred to a new centrifuge tube with a volume of 1.5 mL. The supernatant was exactly the total protein. The total protein was quantitatively analyzed using the BCA quantitative kit manufactured by the Thermo Fisher Corporation. According to the measured protein concentration, an appropriate amount of 5×SDS loading buffer and ddH₂O were added to the protein stock solution, so that the 5×SDS loading buffer was eventually diluted into 1×SDS loading buffer. After thorough mixing, the 1.5 mL of EP tube containing the protein was placed in a metal bath and heated at 100 °C for 10 minutes, and the protein was then taken out for loading electrophoresis. The sample (with a loading volume of 15 µL) was loaded onto a precast gel with a concentration of 4-20wt%, and subjected to an electrophoresis under a voltage condition of 140V for 40min. After completion of the electrophoresis, a 1× transfer solution (containing 20v% anhydrous methanol) was prepared, a piece of PVDF membrane was taken and soaked in anhydrous methanol to activate for 15s, the precast gel was taken out from the electrophoresis tank, the precast gel was fitted with the filter paper, and the transfer rack was finally put into the transfer tank. The membrane was transferred under a voltage of 90V for 120min. After completion of the membrane transfer, the PVDF membrane was taken out and the membrane was washed with TBST for 5min/time, for a total of 2 times. The PVDF membrane was subjected to a sealing treatment for 1 hour with TBST blocking buffer containing 5% skim milk, and the membrane was then washed with TBST for 5min/time, for a total of 3 times. The primary antibody was diluted in different proportions, so that the PVDF membrane was completely immersed in the primary antibody diluent, and shaken slowly overnight at 4 °C. Before incubation with the secondary antibody diluent, the membrane was washed with TBST for 10 min/time, for a total of 5 times, and the secondary antibody was diluted according to a ratio of 1:5,000, the membrane was soaked in the secondary antibody diluent for 1 hour, and the membrane was washed with TBST for 10 min/time, for a total of 3 times. Finally, the luminescent substrate ECL was prepare in the dark according to the ratio of A solution: B solution = 1:1, the PVDF membrane was soaked in ECL for about 5 minutes, then placed in the dark room of the exposure instrument, and the exposure time was determined according to the intensity of the protein band. The intensity of the protein band was subjected to grayscale analysis by using the software Image J.

The present disclosure selected the NCI-H1299 non-small cell lung cancer cell line to test the feasibility of NP-TP53-BIM-PTEN to simultaneously overexpress three tumor suppressor genes in cancer cells. The transfection reagent Lipofectamine 3000 was used, NP-TP53-BIM-PTEN was transfected into NCI-H1299 at different concentrations (0.2-3.2 µg/mL). As a negative control, the multi-gene nanovector NP-EGFP carrying the EGFP gene expression cassette was transfected into NCI-H1299 at a concentration of 2.0 µg/mL. After 48 hours of transfection, the qPCR and Western blotting were used to detect the mRNA expression levels and protein expression levels of cell TP53, BIM and PTEN, respectively.

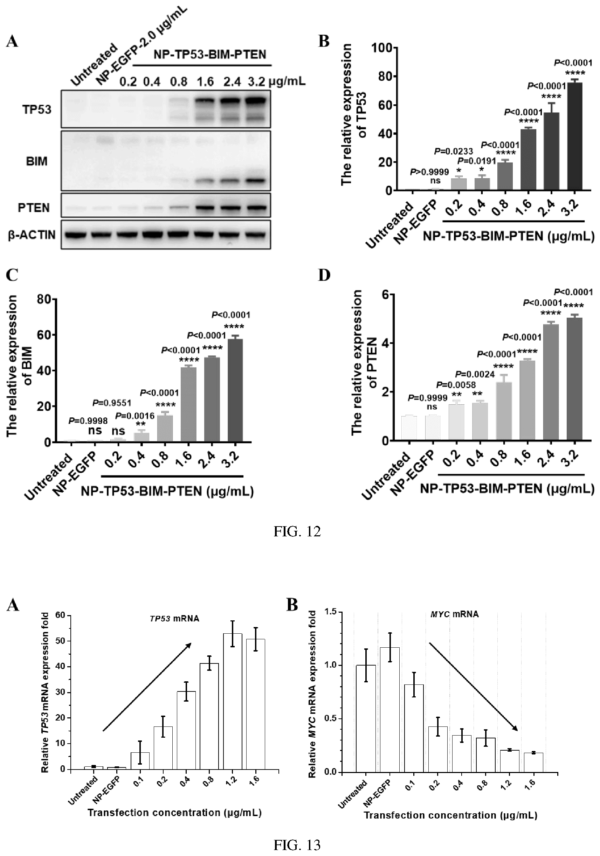

The results are shown in FIG. 11 and FIG. 12, wherein in FIG. 11, A is a graph showing the mRNA expression level of TP53, B is a graph showing the mRNA expression level of BIM, and C is a graph illustrating the mRNA expression level of PTEN; in FIG. 12, A is a Western blotting imaging graph; B is a statistical graph showing the expression level of TP53 protein; C is a statistical graph showing the expression level of BIM protein; and D is a statistical graph illustrating the expression level of PTEN protein.

The results demonstrated that NP-EGFP could not induce the expression of p53, BIM protein and PTEN protein. In cells transfected with NP-TP53-BIM-PTEN, the mRNA and protein expression levels of TP53, BIM and PTEN increased in a dose-dependent manner. As illustrated by FIG. 11, when the transfection concentration of NP-TP53-BIM-PTEN reached 3.2 µg/mL, the mRNA expression quantity of TP53, BIM and PTEN increased by 82,639 times, 685 times and 10 times compared with the untreated group, respectively. As can be seen from FIG. 12, compared with the untreated group, the protein levels of p53, BIM protein and PTEN in NCI-H1299 cells transfected with 3.2 µg/mL NP-TP53-BIM-PTEN increased by 75 times, 57 times and 5 times, respectively. These results demonstrated that NP-TP53-BIM-PTEN can simultaneously overexpress three tumor suppressor genes in the NCI-H1299 non-small cell lung cancer cell line.

### Test Example 3: Multi-gene nanovector NP-TP53-shMYC synchronously overexpresses tumor suppressor genes and silences proto-oncogenes in breast cancer cells

The methods of cell transfection, fluorescent quantitative PCR, and western blot analysis detection were similar to those in Test Example 2, except that:
The present disclosure used breast cancer cells MDA-MB-231 to test the feasibility of NP-TP53-shMYC in synchronously overexpressing tumor suppressor genes and silencing proto-oncogenes in MDA-MB-231 cancer cells. The transfection reagent Lipofectamine 3000 was used, MDA-MB-231 was transfected with NP-TP53-shMYC with different concentrations (0.1-1.6 µg/mL). As a negative control, MDA-MB-231 was transfected with the multi-gene nanovector NP-EGFP carrying the EGFP gene expression cassette at a concentration of 2.0 µg/mL. After 48 hours of transfection, qPCR was used to detect the gene expression levels of TP53 and MYC in cells. Western blotting was used to detect the protein expression levels of TP53 and MYC in cells.

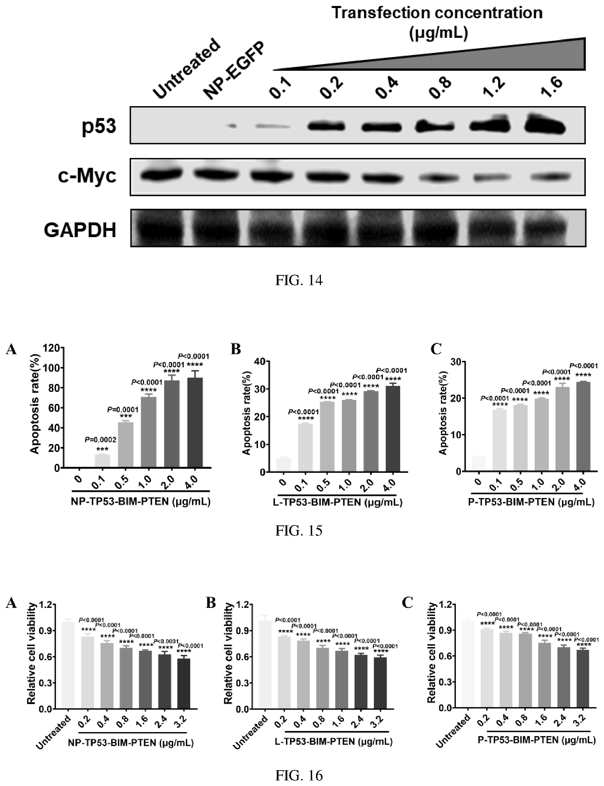

The results are shown in FIG. 13 and FIG. 14. In FIG. 13, A is a statistical graph of the expression level of mRNA for TP53, and B is a statistical graph of the expression level of mRNA for MYC.

The results showed that NP-EGFP did not induce the expression of p53 and silence MYC protein. As illustrated by FIG. 13, in MDA-MB-231 cells, as the transfection dose of NP-TP53-shMYC increased, the TP53 mRNA transcription level continued to increase, especially at a dose of 1.2 µg/mL, the TP53 mRNA transcription multiple increased by about 55 times relative to the negative control group, indicated that NP-TP53-shMYC can overexpress the TP53 gene in breast cancer cells. Relative to the Blank group and NP-EGFP, as the transfection dose of NP-TP53-shMYC increased, the MYC mRNA transcription level continued to decrease, especially at a dose of 1.6 µg/mL, the MYC mRNA transcription multiple decreased by 80% relative to the negative control group, indicated that NP-TP53-shMYC can downregulate MYC gene expression in breast cancer cells by means of RNA interference.

Subsequently, the expression abundance of the corresponding proteins was detected by Western blotting, and the results were consistent with the results of qPCR. As illustrated by FIG. 14, compared with the Blank group and NP-EGFP, when the transfection dose of NP-TP53-shMYC increased, the expression quantity of p53 protein increased progressively in a dose-dependent manner, while the expression quantity of c-Myc protein decreased progressively in a dose-dependent manner.

These results indicate that NP-TP53-shMYC can not only overexpress the tumor suppressor gene TP53 in cancer cells, but also downregulate the abnormally overexpressed proto-oncogene MYC in cancer cells by means of RNA interference, thus realized a combined regulation mode of different gene regulation and control technologies.

**Test Example 4: The multi-gene nanovector NP-TP53-BIM-PTEN with synchronous overexpression of three tumor suppressor genes significantly increased cell apoptosis and proliferation inhibition efficiency**

### 1) Cell viability assay

The CCK8 kit was used to detect cell viability. The specific process was as follows:
a) Plating: NCI-H1299 cells were plated in a 96-well plate, with 1×10⁴ cells per well (125 µL), and the cells were shaken thoroughly to ensure uniform distribution of the cells. Four parallel groups were set up for each experimental group;
b) Transfection: 12-16 hours after cell plating, transfection mixed liquor was prepared according to 5 parallels (×5) per group and transferred to 4 wells respectively. The specific operation was as follows: 1.5µL of Lipofectamine 3000 was diluted with 60.9µL of serum-free medium Opti-MEM, mixed gently with a pipette, and placed at room temperature for 5 minutes; according to the transfection concentration of 1.6µg/mL, 1.6µg of multi-gene nanovector and 1.5µL of P3000 and Opti-MEM were extracted, the volume was 62.5µL in total, added into a 1.5mL centrifuge tube, mixed gently with a pipette, and other different transfection masses were treated with a similar fashion; the Lipofectamine 3000 diluted with Opti-MEM and the multi-gene nanovector diluted with Opti-MEM were mixed with equal volumes, and the mixture was placed at room temperature for 15 minutes; 87.5µL of the original RPMI 1640 complete medium in the 96-well plate was removed, 25µL of transfection mixture was taken and dropwise added into the corresponding wells. The cells were returned to a cell culture incubator at 37 °C and 5% CO₂ and continuously cultured for 48 hours; 37.5 µL of RPMI 1640 complete medium was added to each well of the 96-well plate, maintained 100 µL per well;
c) CCK8 treatment: after 48 hours of culture, 10 µL of CCK8 solution (MedChemExpress) was added to each well, the culture plate was placed in an incubator and incubated for 2 hours, and the absorbance at 450 nm was measured with a microplate reader. After the absorbance at 450 nm was measured, the cell survival rate was calculated according to the formula: cell survival rate = [(As-Ab)/(Ac-Ab)] × 100%, wherein As denoted the absorbance of the experimental well (containing cells, culture medium, CCK-8 solution and drug solution); Ac denoted the absorbance of the control well (containing cells, culture medium and CCK-8 solution, without drug solution); Ab denoted the absorbance of the blank well (containing culture medium and CCK-8 solution, without cells or drug solution).

### 2) Cell apoptosis detection

Annexin V-FITC/PI double staining cell detection kit and flow cytometry was used to detect cell apoptosis. The specific process was as follows:
a) Plating: NCI-H1299 cells were plated in a 12-well plate and the cells were evenly distributed.
b) Transfection: 12-16 hours after cell plating, the multi-gene nanovectors were transfected into NCI-H1299 cells by means of the transfection reagent Lipofectamine 3000. The specific operation was as follows: 2.5 µL of Lipofectamine 3000 was diluted with 97.5 µL of serum-free culture medium Opti-MEM, mixed gently with a pipette, and placed at room temperature for 5 minutes; the calculation was performed according to the transfection concentration of 4 µg/mL, 4 µg of multi-gene nanovector and 2.5 µL of P3000 and Opti-MEM were taken, the total volume was 100 µL, the materials were added into a 1.5mL centrifuge tube, mixed gently with a pipette, and other different transfection masses were treated with a similar manner; the Lipofectamine 3000 diluted with Opti-MEM and the multi-gene nanovector diluted with Opti-MEM were mixed with the equal volumes, the mixture was placed at room temperature for 15 minutes; the original RPMI 1640 complete culture medium in the 12-well plate was discarded, 300 µL of Opti-MEM culture medium was added, and 200 µL of the mixed liquor was evenly and dropwise add into the corresponding wells, the 12-well plate was gently shaken such that the mixed liquor was evenly distributed. The cells were returned to a cell culture incubator at 37 °C and 5v% CO₂ and cultured for 4 hours, RPMI 1640 complete medium was then replaced with at a volume of 1 mL/well and continuously cultured for 48 hours.
c) Cell collection: a pipette was used to collect the supernatant in a 15mL centrifuge tube. After centrifugation, the cells were washed once with PBS. At the same time, 200µL of trypsin was added into each well to digest the adherent cells. After digestion for 2 minutes, 1mL of RPMI 1640 complete medium was added. Finally, the adherent digested cell suspension was mixed with the supernatant collected by PBS resuspending cell suspension, the mixture was subjected to a centrifugation at the rotational speed of 2,000rpm under room temperature for 3-5min, the supernatant was discarded and the cells were collected. The cells were resuspended once with pre-cooled 1×PBS, centrifuged at the rotational speed of 2,000rpm for 3-5min, and the cell pellet was collected;
d) Dye incubation: 300 µL of 1× Binding Buffer was added, the suspended cells were mixed gently and uniformly, 5 µL of Annexin V-FITC dye was added, and incubation was performed in the dark at room temperature for 15 minutes; 5 minutes before loading, 5 µL of PI dye was added and 200 µL of 1× Binding Buffer was replenished.
e) Fluorescence signal collection: cell apoptosis condition was detected by a flow cytometry (LSRFortessa, BD), and the software FlowJo-V10 was used to analyze the cell apoptosis condition.

The present disclosure selected the NCI-H1299 non-small cell lung cancer cell line to test whether simultaneous overexpression of three tumor suppressor genes in cancer cells, NP-TP53-BIM-PTEN will increase the apoptotic activity of cells. After 48 hours of transfection, FITC-labeled Annexin V and PI flow cytometry were used to detect cell apoptosis condition.

The results are shown in FIG. 15 and FIG. 16, wherein in FIG. 15, A is a statistical graph illustrating the apoptosis rate of NCI-H1299 cancer cells at different concentrations of NP-TP53-BIM-PTEN; B is a statistical graph showing the apoptosis rate of NCI-H1299 cancer cells at different concentrations of L-TP53-BIM-PTEN; C is a statistical graph illustrating the apoptosis rate of NCI-H1299 cancer cells at different concentrations of P-TP53-BIM-PTEN. In FIG. 16, A illustrates a statistical graph of the relative cell viability of NCI-H1299 cancer cells at different concentrations of NP-TP53-BIM-PTEN; B illustrates a statistical graph of the relative cell viability of NCI-H1299 cancer cells at different concentrations of L-TP53-BIM-PTEN; C illustrates a statistical graph of the relative cell viability of NCI-H1299 cancer cells at different concentrations of P-TP53-BIM-PTEN.

The results demonstrated that NP-TP53-BIM-PTEN induced significant apoptosis in NCI-H1299 cells, and its apoptosis induction rate was dose escalation-dependent. As can be seen from Figure 15A, within the transfection concentration range (0.1-4.0 µg/mL), the apoptosis rate induced by NP-TP53-BIM-PTEN was within the range of 13.55%-94.90%; among them, the average apoptosis induction rate of cells treated with 4.0 µg/mL NP-TP53-BIM-PTEN was close to 95%; and as illustrated by FIG. 15B and FIG. 15C, the apoptosis rates induced by L-TP53-BIM-PTEN and P-TP53-BIM-PTEN were relatively weak, and the apoptosis induction rates were maintained at the ranges of 14.88%-32.90% and 3.49%-24.87%, respectively. When the transfection concentration was 4.0 µg/mL, the cell apoptosis rate induced by NP-TP53-BIM-PTEN was 2.9-fold and 3.7-fold higher than that of L-TP53-BIM-PTEN and P-TP53-BIM-PTEN, respectively.

After transfection for 48 hours, the cell proliferation inhibitory activity was further measured by using a cell counting kit 8 (CCK8). The results showed that, as illustrated by FIG. 16A, NP-TP53-BIM-PTEN can efficiently inhibit the proliferation of NCI-H1299 cells. At a transfection concentration of 3.2 µg/mL, the inhibition rate of NP-TP53-BIM-PTEN on NCI-H1299 cell proliferation may reach 42.00% ± 4.40%; and as can be seen from FIG. 16B and FIG. 16C, the inhibition rates of L-TP53-BIM-PTEN and P-TP53-BIM-PTEN on NCI-H1299 cell proliferation were 40.69% ± 3.77% and 32.92% ± 1.65%, respectively, which were significantly lower than the cell proliferation inhibition rate of NP-TP53-BIM-PTEN.

These results demonstrate that the three tumor suppressor genes (TP53, BIM, and PTEN) delivered by multi-gene nanovectors can be simultaneously expressed in NCI-H1299 and significantly enhance the cell apoptosis induction rate, and also greatly reduce cell proliferation activity, indicated that restoring the expression ability of multiple tumor suppressor genes in cancer cells will be conducive to improving the killing toxicity of gene therapy to cancer cells.

### Test Example 5: Multigene nanovector NP-TP53-shMYC that simultaneously overexpresses tumor suppressor genes and silences proto-oncogenes significantly increases cell apoptosis activity

The detection method of cell apoptosis was similar to that of test example 4, except that:
The present disclosure selected the breast cancer cells MDA-MB-231 to test whether the NP-TP53-shMYC that simultaneously overexpressed a tumor suppressor gene (TP53) and produced shRNA to silence a proto-oncogene (MYC) in cancer cells can increase cell apoptosis activity. MDA-MB-231 cells were transfected with different concentrations of NP-TP53-shMYC (0-2.0 µg/mL), and 48 hours after transfection, the FITC-labeled Annexin V and the PI flow cytometry were used to detect the cell apoptosis condition, wherein NP-EGFP was used as a negative control, and NP-TP53 and NP-shMYC were used as single gene controls for comparative analysis.

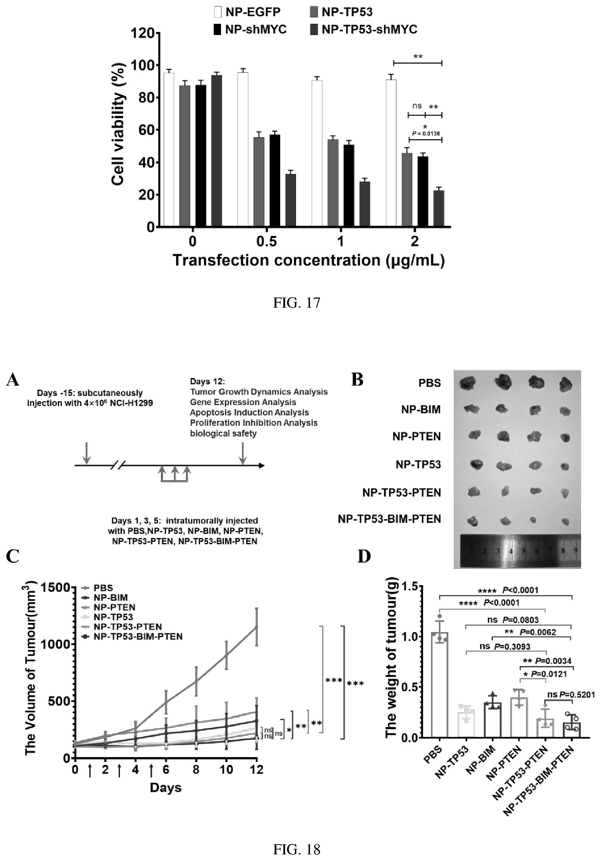

The results are shown in Figure 17. The analysis results of flow cytometer demonstrated that NP-EGFP could not induce apoptosis of MDA-MB-231 cells, while NP-TP53-shMYC could significantly induce apoptosis of MDA-MB-231 cells, and the apoptosis induction rate was dose escalation-dependent.

As can be seen from FIG. 17, when the transfection concentration of NP-TP53-shMYC was 2.0 µg/mL, the average apoptosis rate of MDA-MB-231 cells was close to 77.4%; when the average apoptosis rates of MDA-MB-231 cells induced by NP-TP53 and NP-shMYC were 54.1% and 56.3%, respectively, indicated that the simultaneous targeting of TP53 gene and MYC gene induced stronger apoptosis activity in cancer cells than that induced by single gene targeting. The bidirectional gene regulation of TP53 overexpression and MYC downregulation by RNA technology by means of multi-gene nanovectors was conducive to enhancing the sensitivity of cancer cell apoptosis signaling pathways, thereby showed stronger apoptosis inducing activity.

### Test Example 6: Multigene nanovector NP-TP53-BIM-PTEN with synchronous overexpression of three tumor suppressor genes enhances antitumor effect in xenograft tumor mice

### 1) Construction of xenograft tumor mouse model

The animal model used to construct the xenograft tumor was a BALB/c nude male mouse aged 6-8 weeks. The specific operation was as follows: After the mice were purchased from Beijing Weitong Lihua Company, they were first cultured for one week under sterile conditions. The mouse culture conditions needed to meet the following requirements: the sterile operation principle was strictly followed, the mice were fed in different cages in a laminar flow chamber, all the utensils used were sterilized at high temperature, the bedding, feed and drinking water were sterilized, the laboratory environment temperature was within the range of 18 °C -28 °C, the relative humidity was within the range of 40%-60%, the mice were free to ingest and drink water, the lighting was alternating for 12h (8:00-20:00), the bedding was kept dry, and the bedding was changed twice a week. When the mice were in good condition, NCI-H1299 cells (3×106 ) resuspended in 100µL pre-cooled PBS were injected subcutaneously in the right lower abdomen of mice. 15 days after the injection of the cells, a tumor mass of about 100mm³ was formed in the right lower abdomen of the mice, indicated that the mouse model was successfully constructed.

### 2) Multi-gene nanovector delivery

The successfully modeled mice were randomly divided into 6 groups, with 4 mice in each group, and treated by intratumoral administration. The multi-gene nanovector was delivered by liposome 2000 (Lipofectamine 2000), and each 100µL injection volume contained 45µL of Lipofectamine 2000, the dosage of the multi-gene nanovector was determined according to the weight of mice, and the intratumoral administration was performed at 2.25mg/kg, once every two days, for a total of 3 times. The specific grouping and dosing schedule were as follows: negative control group (100µL PBS), multi-gene nanovector test group (100µL multi-gene nanovector, 45µg).

### 3) Observation and recording of tumor growth status

After administration, the tumor volume, weight and body weight of the mice were observed and recorded every day until the mice were treated. The tumor volume and body weight change trend graphs were drawn according to the weight volume and body weight changes of mice on different days.

### 4) Molecular marker tracing related to tumor growth and apoptosis

After the multi-gene nanovector entered the tumor tissue, whether the multi-gene nanovector can effectively regulate and control the expression of related genes and induce cell apoptosis and cell proliferation inhibition in the tumor tissue required further detection of changes in molecular markers related to growth and apoptosis in the tumor tissue. The detection methods of molecular markers related to tumor growth and apoptosis mainly included fluorescent quantitative PCR, Western blotting, TUNEL apoptosis analysis and immunohistochemistry analysis. The specific operation process of each method was as follows:

### a) Fluorescent quantitative PCR

After the tumor tissue was excised, the tissue was ground by using a high-throughput tissue grinder, and total RNA was extracted by using an animal tissue/cell RNA extraction kit (manufactured by Jiangsu CoWin Bioscience Co., Ltd.). The reverse transcription reaction system was: 1µg of RNA, 1µL of gDNA Remover, 4µL 5×TransScript^{®} All-in-One SuperMix for qPCR, supplemented with Nuclease-free H₂O to 1µL. The reverse transcription reaction process was: 42°C, 15min - 85°C, 5s. Preparation of the fluorescent quantitative PCR reaction system: 1µL of cDNA, 0.5µL of quantitative primer (10µM), 10µL of 2×PerfectStart^{®} Green qPCR SuperMix (TransGen Biotech), supplemented with Nuclease-free H₂O to 20µL. The fluorescence quantitative PCR program was 94°C, 5min - (94°C, 5s - 58°C, 20s - 72°C, 5min) × 40cycles. The relative expression quantity analysis of different genes was calculated using the 2^{-ΔΔCq} method.

### b) Western blot analysis

After the tumor tissue was excised, the tissue was ground by using a high-throughput tissue grinder, an appropriate volume of RIPA lysis buffer was added. After the tumor tissue was fully lysed, the tissue fluid was transferred to a 1.5 mL centrifuge tube and placed in a 4 °C centrifuge for centrifugation at rotational speed of 12,000 rpm for 10 minutes. The supernatant was transferred to a new centrifuge tube with a volume of 1.5 mL, and the supernatant was exactly the total protein. The total protein was quantitatively analyzed using the BCA quantitative kit manufactured by the Thermo Fisher Corporation. According to the measured protein concentration, an appropriate amount of 5 ×SDS loading buffer and ddH₂O were added to the protein stock solution, so that the 5 ×SDS loading buffer was eventually diluted into 1 ×SDS loading buffer. After thorough mixing, the 1.5 mL of EP tube containing the protein was placed in a metal bath and heated at 100 C for 10 minutes, and the protein was then taken out for loading electrophoresis. The sample (with a loading volume of 15 µL) was loaded onto a precast gel with a concentration of 4-20wt%, and subjected to an electrophoresis under a voltage condition of 140V for 40min. After completion of the electrophoresis, a 1 × transfer solution (containing 20% anhydrous methanol) was prepared, a piece of PVDF membrane was taken and soaked in anhydrous methanol to activate for 15s, the precast gel was taken out from the electrophoresis tank, the precast gel was fitted with the filter paper, and the transfer rack was finally put into the transfer tank. The membrane was transferred under a voltage of 90V for 120min. After completion of the membrane transfer, the PVDF membrane was taken out and the membrane was washed with TBST for 5min/time, for a total of 2 times. The PVDF membrane was subjected to a sealing treatment for 1 hour with TBST blocking buffer containing 5% skim milk, and the membrane was then washed with TBST for 5min/time, for a total of 3 times. The primary antibody was diluted in different proportions, so that the PVDF membrane was completely immersed in the primary antibody diluent, and shaken slowly overnight at 4 °C. Before incubation with the secondary antibody diluent, the membrane was washed with TBST for 10 min/time, for a total of 5 times, and the secondary antibody was diluted according to a ratio of 1:5,000, the membrane was soaked in the secondary antibody diluent for 1 hour, and the membrane was washed with TBST for 10 min/time, for a total of 3 times. Finally, the luminescent substrate ECL was prepare in the dark according to the ratio of A solution: B solution = 1:1, the PVDF membrane was soaked in ECL for about 5 minutes, then placed in the dark room of the exposure instrument, and the exposure time was determined according to the intensity of the protein band. The intensity of the protein band was subjected to grayscale analysis by using the software Image J.

### c) TUNEL apoptosis assay

The tumor tissue was collected, placed in a paraffin tissue embedding box, immersed in 4% paraformaldehyde for fixation for 48 hours, and dehydrated step by step according to different concentration gradients of ethanol, 70% ethanol 1h - 70% ethanol 2h - 80% ethanol 30min - 90% ethanol 30min - 95% ethanol (I) 30min - 95% ethanol (II) 1h - 100% ethanol (I) 30min - 100% ethanol (II) 40min - xylene (I) 20min - xylene (II) 35min - wax tank (I) 1h - wax tank (II) 1h. The dehydrated tumor tissue was embedded in a paraffin embedding machine to form a paraffin tissue block, and the paraffin tissue block was sliced with a paraffin slicer, and 4µm thick tumor tissue sections were cut and placed in a slice box for storage at room temperature. Subsequently, the sections were placed in xylene (I) for 15 min, xylene (II) for 15 min, anhydrous ethanol (I) for 5 min, anhydrous ethanol (II) for 5 min, 85% alcohol for 5 min, 75% alcohol for 5 min, and washed with distilled water. Finally, the paraffin sections were dewaxed and placed in water. After the sections were slightly dried, a circle was drawn around the tissue with a tissue pen (to prevent the liquid from flowing away), and the proteinase K working solution was dropwise added into the circle to cover the tissue, and incubated in a 37 °C incubator for 15-30 min. The slides were placed in PBS (pH 7.4) and washed on a decolorizing shaker for 3 times, each time for 5 min. After the sections were slightly dried, the membrane breaking working solution was dropwise added into the circle to cover the tissue, and incubated at room temperature for 20 min. The slides were placed in PBS (pH 7.4) and swayed and washed on a decolorizing shaker for 3 times, each time for 5 min. According to the number of slides and the size of the tissue, appropriate amounts of reagent 1 (TdT) and reagent 2 (dUTP) in the TUNEL kit were mixed in a ratio of 1:9 and kept away from light for later use. The prepared TUNEL was added to the circle to cover the tissue, the slices were placed flat in a humid box, incubated in a constant temperature incubator at 37 °C for 2h, and a small amount of water was added into the humid box to maintain humidity. The slices was washed with PBS (pH7.4) for 3 times, 5min each time. After PBS was removed, the DAPI staining solution was dropwise added into the circle and incubated in the dark at room temperature for 10min. The slides were placed in PBS (pH7.4) and shaken on a decolorizing shaker to wash 3 times, 5min for each time. After the slices were slightly dried, they were sealed with anti-fluorescence quenching sealing agent. The slices were observed under a fluorescence microscope and images were collected. The DAPI ultraviolet excitation wavelength was within the range of 330-380nm, the emission wavelength was 420nm, and blue light was emitted; the FITC excitation wavelength was within the range of 465-495nm, the emission wavelength was within the range of 515-555nm, and green light was emitted; the CY3 excitation wavelength was within the range of 510-560nm, and the emission wavelength was 590nm, and red light was emitted.

### d) Immunohistochemical analysis of proliferation marker Ki67

The paraffin sections were placed in xylene (I) for 20 min, xylene (II) for 20 min, xylene (III) for 15 min, anhydrous ethanol (I) for 5 min, anhydrous ethanol (II) for 5 min, 85% alcohol for 5 min, 75% alcohol for 5 min, and washed with distilled water. The tissue sections were placed in a microwave oven filled with citric acid antigen retrieval buffer (pH 6.0) or EDTA antigen retrieval buffer (pH 9.0) for antigen retrieval, treated with medium heat, medium-high heat, and high heat for 5 min respectively. After natural cooling, the slides were in PBS (pH 7.4) and shaken, washed and soaked for 3 times, 5 min for each time.

3% hydrogen peroxide solution was placed in the slices, which were incubated in the dark at room temperature for 25 minutes, the slides were placed in PBS (pH 7.4), shaken, washed and soaked for 3 times, 5 minutes for each time. After the slices were slightly dried, a circle was drawn around the tissue with a tissue pen, goat serum was added into the tissue circle to evenly cover the tissue, and sealed at room temperature for 30 minutes. The blocking buffer was gently shaken off, a primary antibody diluted with a ratio of 1:200 was added to the slices, and the slices were placed flat in a humid box and incubated at 4 °C overnight. The slices were placed in PBS (pH 7.4), and shaken and washed for 3 times, 5 minutes for each time. After the slices are slightly dried, a secondary antibody (HRP labeled) of the corresponding species to the primary antibody was dropwise added in the circle to cover the tissue and incubated at room temperature for 1 hour. The slices were place in PBS (pH 7.4), shaken and washed for 3 times, 5 minutes for each time. After the slices are slightly dried, the freshly prepared DAB color development solution was added to the circle, the color development time under a microscope was controlled, positive was brown-yellow, and the slices were rinsed with tap water to stop color development. Hematoxylin counterstaining was performed for about 5-10 minutes, rinsed with tap water, differentiated with 1% hydrochloric acid alcohol for a few seconds, rinsed with tap water, then returned to blue with 1% ammonium hydroxide aqueous solution for 1 minute, further rinsed with tap water. The slices were placed in 75% alcohol for 5 minutes, 85% alcohol for 5 minutes, anhydrous ethanol (I) for 5 minutes, anhydrous ethanol (II) for 5 minutes, and xylene (I) for 5 minutes in sequence to dehydrate and become transparent. The slices were taken out of xylene and dried slightly, and sealed with neutral gum. After sealing, photos were collected under a microscope. Hematoxylin stained the cell nucleus blue, and DAB showed positive expression as brown-yellow. A semi-quantitative method was used for statistical analysis, and a staining score (score 0-12) standard was established: 1. Scoring was performed according to the intensity of positive staining (weak: 1 point; mild: 2 points; strong: 3 points); 2. Scoring was performed according to the proportion of positive cells of interest (0%, 0 point; <25%, 1 point; 26-50%, 2 points; 51-75%, 3 points; >76%, 4 points). Statistics were performed according to the established scoring system, with 4 mice in each group, and 2 visual fields for each mouse were counted.

The present disclosure selected a tumor-bearing mouse model transplanted with NCI-H1299 non-small cell lung cancer cell line and adopted an intratumor injection method to test the anti-tumor therapeutic effect of NP-TP53-BIM-PTEN.

FIG. 18 shows an evaluation of the in vivo anti-tumor therapeutic effect of multi-gene nanovector, wherein A illustrates the therapeutic regimen; B shows the tumor image of the mouse 12 days after administration, PBS indicates that the mice are administered with PBS containing Lipofectamine 2000; NP-TP53 indicates that the mice are administered NP-TP53 with the help of Lipofectamine 2000; NP-BIM indicates that the mice are administered NP-BIM with the help of Lipofectamine 2000; NP-PTEN indicates that the mice are administered NP-PTEN with the help of Lipofectamine 2000; NP-TP53-PTEN indicates that the mice are administered NP-TP53-PTEN with the help of Lipofectamine 2000; NP-TP53-BIM-PTEN indicates that the mice are administered NP-TP53-PTEN with the help of Lipofectamine 2000; C illustrates the tumor growth curve within 12 days of administration. The experimental group is administered with a dose of 2.25 mg/kg every 2 days (1st, 3rd, and 5th days), for a total of 3 treatments. The arrows indicate the time of administration; D illustrates a statistical chart of tumor weights in different treatment groups on the 12th day.

As can be seen from the therapeutic regimen in A of FIG. 18, the successfully modeled mice were randomly divided into 6 groups, 4 mice in each group, and treated by intratumoral administration. The multi-gene nanovector was delivered by Lipofectamine 2000, and 45 µL of Lipofectamine 2000 was contained in each 100 µL of injection volume. The dosage of the multi-gene nanovector was determined according to the weight of said mouse, and intratumoral administration was performed at 2.25 mg/kg, once every two days, for a total of 3 times. The specific grouping and administration scheme were as follows: negative control group (100 µL of PBS), multi-gene nanovector test group (100 µL of multi-gene nanovector, 45 µg). The anti-tumor treatment test was arranged as follows: PBS (negative control group), NP-TP53, NP-BIM, NP-PTEN, NP-TP53-PTEN, NP-TP53-BIM-PTEN.

As can be seen from B of FIG. 18, compared with the in vivo negative control, NP-TP53-PTEN and NP-TP53-BIM-PTEN showed significant anti-tumor activity, which was consistent with the anti-tumor effect in vitro.

As can be seen from C and D of FIG. 18, compared with the negative control group, NP-TP53, NP-BIM and NP-PTEN also showed anti-tumor activity, but their anti-tumor activity was lower than that of the NP-TP53-PTEN and NP-TP53-BIM-PTEN treatment groups. The average tumor volume and tumor weight of mice in the NP-TP53-BIM-PTEN group on the 12th day of administration were reduced by 84.7% and 85.2% respectively compared with the PBS group. Similarly, on the 12th day of administration, the average tumor volume and tumor weight of mice in the NP-TP53-PTEN group were reduced by 80.8% and 81.6% respectively compared with the PBS group. The average tumor volume and tumor weight of mice in the NP-TP53 group were reduced by 76.9% and 76.0% respectively compared with the PBS group. The average tumor volume and tumor weight of mice in the NP-BIM group were reduced by 71.4% and 66.6% respectively compared with the PBS group. The average tumor volume and tumor weight of mice in the NP-PTEN group were reduced by 64.7% and 62.0% respectively compared with the PBS group. The average tumor volume and tumor weight of the gene/multi-gene nanovector group carrying two or three gene expression cassettes were much lower than those of the gene/multi-gene nanovector group carrying a single gene expression cassette. Compared with NP-TP53, NP-BIM and NP-PTEN, the average tumor volume of NP-TP53-BIM-PTEN was reduced by 33.7%, 46.5% and 56.6%, respectively. Compared with NP-TP53, NP-BIM and NP-PTEN, the tumor weight of mice was reduced by 38.5%, 55.9% and 61.2%, respectively. Compared with NP-TP53 and NP-PTEN, the average tumor volume of NP-TP53-PTEN was reduced by 16.7% and 45.4%, respectively. Compared with NP-TP53 and NP-PTEN, the tumor weight of mice was reduced by 23.2% and 51.6%, respectively. The average tumor volume and tumor weight of mice in the NP-TP53-BIM-PTEN group were reduced by 20.4% and 19.9%, respectively, compared with the NP-TP53-PTEN group.

These results indicate that the three tumor suppressor genes (TP53, BIM, PTEN) delivered by multi-gene nanovectors can also be simultaneously expressed in NCI-H1299 xenograft tumors and exhibit the single gene overexpression-mediated anti-tumor therapeutic effects.

The present disclosure further uses qPCR and Western blotting techniques to quantitatively analyze the expression levels of three tumor suppressor genes to verify whether the inhibition of tumor growth is related to the restoration of TP53, BIM and PTEN gene expression in the tumor.

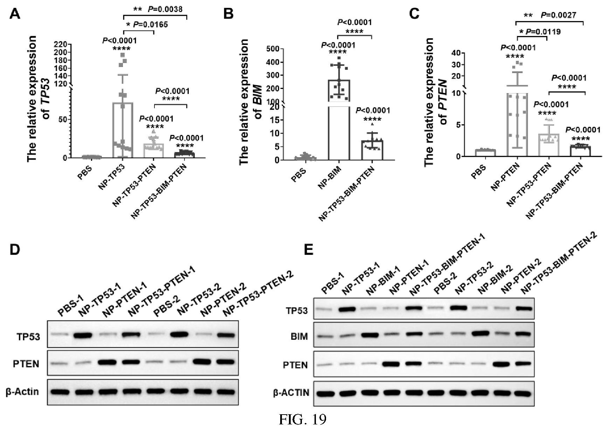

The results are shown in FIG. 19, wherein A illustrates the qPCR detection of TP53 mRNA level in tumor tissue on day 12; B shows the qPCR detection of BIM mRNA level in tumor tissue on day 12; C illustrates the qPCR detection of PTEN mRNA level in tumor tissue on day 12; D and E illustrate Western blotting detections of TP53, BIM, and PTEN protein levels in tumor tissue on day 12.

As can be seen from A of FIG. 19, compared with the PBS group, qPCR detection on day 12 after administration showed that the expression of these genes in the NP-TP53-BIM-PTEN group and the NP-TP53-PTEN group was upregulated. The TP53 mRNA expression levels in the NP-TP53-PTEN group and the NP-TP53-BIM-PTEN group were upregulated by 16.9 times and 5.5 times, respectively.

As can be seen in B of FIG. 19, the expression of BIM mRNA in the NP-TP53-BIM-PTEN group was upregulated by 7.0 times.

As can be seen from C of FIG. 19, the PTEN mRNA expression levels in the NP-TP53-PTEN group and the NP-TP53-BIM-PTEN group were upregulated by 3.5 times and 1.6 times, respectively.

As illustrated by D and E of FIG. 19, the p53 protein expression levels in the NP-TP53-PTEN group and the NP-TP53-BIM-PTEN group were upregulated by 13.7 and 11.2 times, respectively, which were consistent with the mRNA levels on day 12 after administration. The BIM protein expression level in the NP-TP53-BIM-PTEN group was upregulated by 4.3 times. The PTEN protein expression levels in the NP-TP53-PTEN group and the NP-TP53-BIM-PTEN group were upregulated by 10.2 and 7.7 times, respectively.

These results indicate that NP-TP53-BIM-PTEN can simultaneously produce the three designed proteins in a xenograft tumor model.

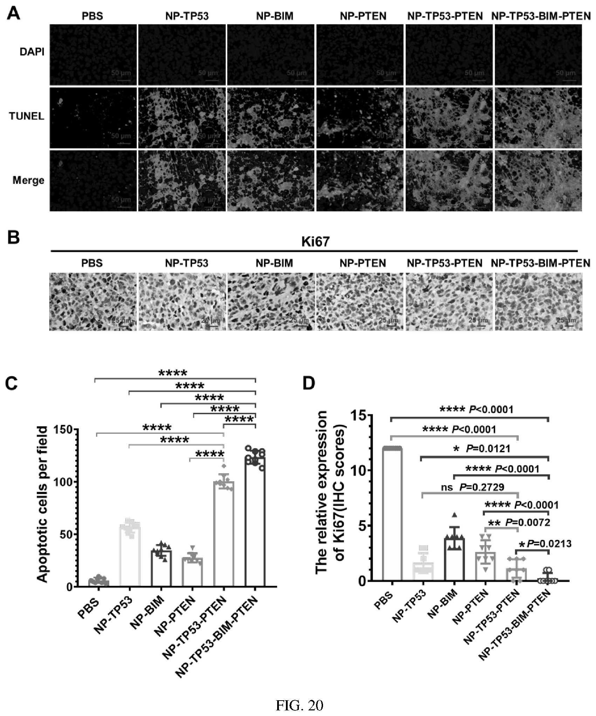

The present disclosure further uses TUNEL and immunohistochemical technology to quantitatively analyze apoptosis markers and cell proliferation markers Ki67, in order to verify whether tumor growth inhibition is directly related to an increase of apoptosis signal markers and cell proliferation markers in tumor tissues. The results are shown in FIG. 20, wherein A illustrates the TUNEL method for detecting the apoptosis rate of NCI-H1299 xenograft tumor cells on the 12th day after administration, and DAPI and DeadEndTM fluorescent TUNEL system confocal microscope are used for fluorescence imaging; B shows the immunohistochemical method for detecting the expression of NCI-H1299 xenograft tumor cell proliferation marker Ki67; C illustrates a statistical analysis of the apoptosis ratio of tumor tissues treated with different drugs; D shows a semi-quantitative statistical analysis of the abundance of Ki67 in tumor tissues treated with different drugs.

As illustrated by A and C of FIG. 20, on the 12th day after administration, the detection results of the TUNEL assay showed that the NP-TP53-BIM-PTEN group had the highest apoptosis rate, which was about 19.4 times that of the PBS group, slightly higher than the NP-TP53-PTEN group. The apoptosis rate of the NP-TP53-PTEN group was about 15.9 times that of the PBS group. For the gene multi-gene nanovector carrying a single gene expression cassette, the apoptosis rates of the NP-TP53 group, NP-PTEN group, and NP-BIM group were 8.9 times, 4.4 times, and 5.5 times higher than those of the PBS group, respectively.

As illustrated by B and D of FIG. 20, on the 12th day after administration, Ki67 antibody immunohistochemistry was used to detect the proliferation of cells with different treatments. According to the staining score (0-12) standard of 4 mice per group and 2 fields of view per mouse. The results showed that compared with the PBS group, the expression of Ki67 protein in the NP-TP53, NP-BIM, NP-PTEN, NP-TP53-PTEN, and NP-TP53-BIM-PTEN groups was downregulated by 86.4%, 67.7%, 78.1%, 90.6%, and 97.9%, respectively.

The above results indicate that the synchronous overexpression of three tumor suppressor genes mediated by multigene nanovectors (NP-TP53, NP-BIM, and NP-PTEN) shows better effects on tumor cell proliferation inhibition in xenograft tumors compared with multigene nanovectors carrying single genes.

### Test Example 7: Multigene nanovector NP-TP53-shMYC that overexpresses tumor suppressor genes and silences proto-oncogenes enhances the anti-tumor effect in transplanted tumor mice

The construction of xenograft tumor mouse model, multi-gene nanovector delivery, observation and recording of tumor growth status, and evaluation methods of tumor growth and apoptosis-related molecular marker tracing in Test Example 7 were similar to those of Test Example 6, except that:
The present disclosure selected a tumor-bearing mouse model transplanted with MDA-MB-231 (5-week female BALB/c nude mice) and used an intratumoral injection method to test the anti-tumor therapeutic effect of NP-TP53-shMYC. The successfully modeled mice were randomly divided into 7 groups, 4 mice in each group, and treated by intratumoral administration. The multi-gene nanovector was delivered by Lipofectamine 2000, and each 100 µL injection volume contained 45 µL of Lipofectamine 2000. The dosage of the multi-gene nanovector was determined according to the weight of the mouse, and the intratumoral administration was performed at 2.25 mg/kg, once every two days, for a total of 3 times. The specific grouping and dosage regimen were as follows: negative control group (100 µL PBS), multi-gene nanovector test group (100 µL multi-gene nanovector, 45 µg). The anti-tumor treatment test was set up with the following groups: PBS (negative control group), L-TP53-shMYC (added with Lipofectamine 2000), L-TP53-shMYC(-) (without Lipofectamine 2000), NP-TP53 (added with Lipofectamine 2000), NP-shMYC (added with Lipofectamine2000), NP-TP53-shMYC (added with Lipofectamine 2000) and NP-TP53-shMYC(-) (without Lipofectamine 2000).

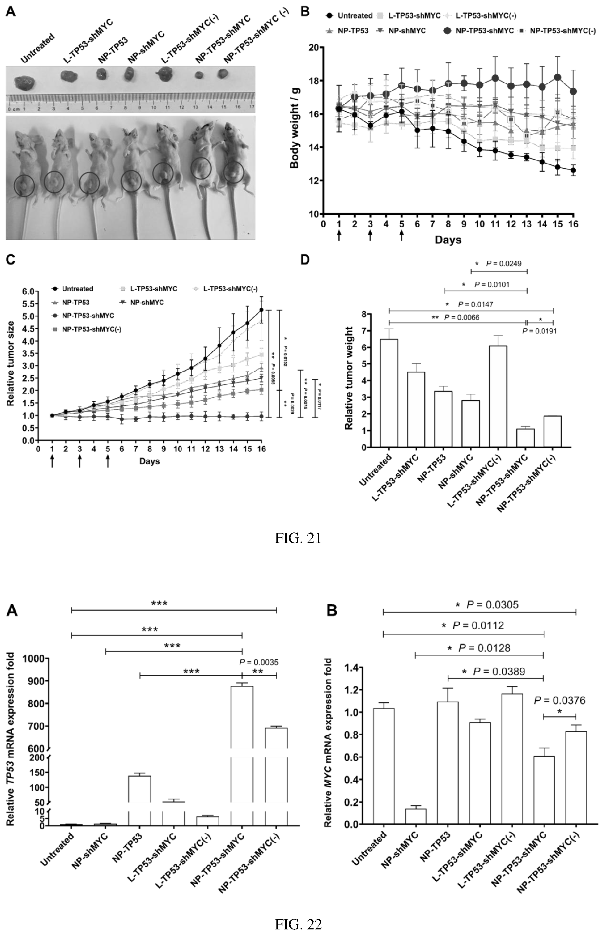

The results are shown in FIG. 21, wherein in FIG. 21, A illustrates a photo of the tumor excised and treated mice on the 16th day after administration; B shows a record of the weight changes of each group of MDA-MB-231 tumor-bearing mice within 16 days after administration (black arrows indicate the time point of each treatment); C illustrates a record of the changes in the relative volume of the tumor within 16 days after administration of each group of mice (black arrows indicate the time point of each treatment); D shows the relative tumor weight of different treatment groups on the 16th day after administration. The intratumoral injection dose for each mouse was 2.25 mg/kg, injected once every 2 days, for a total of 3 treatments (1st, 3rd, and 5th days).

As can be seen from A and B of FIG. 21, consistent with the anti-tumor effect in vitro, NP-TP53-shMYC exhibited better anti-tumor effect in vivo, with anti-tumor activity higher than all other test groups, and its mice did not experience a significant decrease in body weight.

As can be seen from C and D of FIG. 21, tumor growth in mice treated with NP-TP53-shMYC was basically stagnant, and the tumor volume did not change much within 16 days. Compared with the untreated group, on the 16th day after administration, its tumor volume was reduced by 85.2%, and the tumor weight was also reduced by 82.9%, compared with the untreated group. The therapeutic effect of this treatment group was also much higher than all other treatments, especially the single gene therapy groups NP-TP53 and NP-shMYC. In NP-TP53-treated mice, the tumor volume and tumor weight decreased by 47.9% and 48.2% respectively on the 16th day compared with the untreated group, while the tumor volume and weight of NP-shMYC-treated mice were reduced by 57.6% and 56.7% respectively compared with the untreated group. In contrast, L-TP53-shMYC (-) had no effect on tumor volume and tumor weight. Only when Lipofectamine 2000 was added, the anti-tumor activity of L-TP53-shMYC was slightly improved, however, it was still much lower than NP-TP53-shMYC.

This result indicates that linear DNA-based in vivo therapy heavily relies on transfection agents to reduce drug resistance of the nuclease, but the serum stability advantage of multi-gene nanovectors is well reflected in the in vivo therapy. For example, in the case of transfection-assisted delivery of Lipofectamine 2000, NP-TP53-shMYC(-) has higher anti-tumor activity than NP-TP53 and NP-shMYC, but its anti-tumor activity is still lower than that of NP-TP53-shMYC delivered with the assistance of Lipofectamine 2000. It reveals that multi-gene nanovectors have a certain degree of tumor tissue penetration ability, and can autonomously enter cells to regulate related gene expression and cell physiological activity without relying on liposomes. Especially under the premise of synchronous regulation of the two key genes TP53 and MYC, the multi-gene nanovectors can exert a therapeutic effect beyond single gene regulation, providing a reference for the subsequent optimization of different gene combinations.

The present disclosure further used qPCR and Western blotting techniques to quantitatively analyze the expression levels of TP53 and MYC in tumor tissues to verify whether the inhibition of tumor growth is directly related to the restored expression of TP53 and decreased expression of MYC in the tumor. The tumors in the mice were removed 48 hours after the third administration, and the tumors were broken and lysed for qPCR analysis. The tumors in the mice were removed 48 hours after the third administration, and the tumors were broken and lysed for Western blotting analysis.

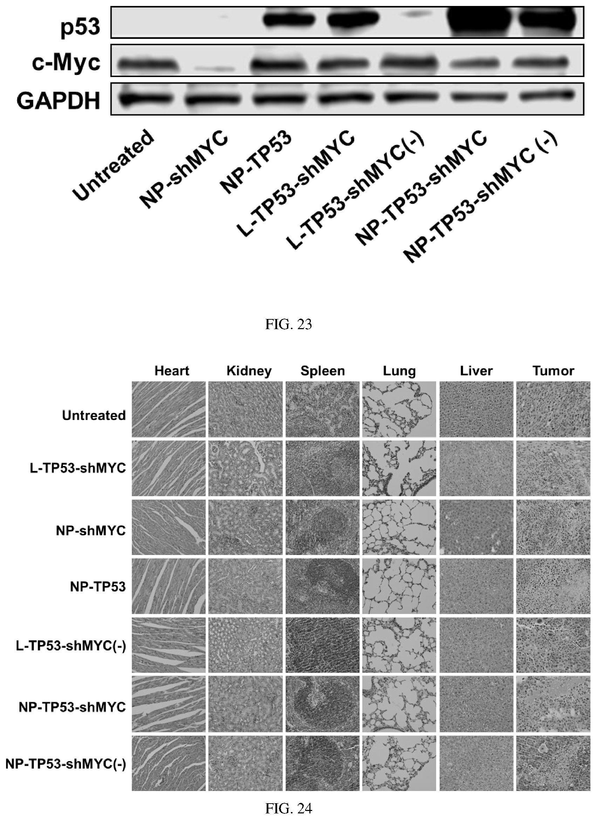

The results are shown in FIG. 22 and FIG. 23, wherein in FIG. 22, A illustrates the relative expression quantity analysis of the TP53 gene; B shows the relative expression quantity analysis of the MYC gene.

As illustrated by A of FIG. 22, 48 hours after the third administration, the tumor in the mouse was removed, broken and lysed for qPCR and western blotting analysis. For the TP53 gene, compared with the negative control, the NP-shMYC multi-gene nanovector had no effect on TP53 expression, while NP-TP53 could increase the expression of the TP53 gene in tumor cells. L-TP53-shMYC can only increase the expression level of TP53 gene in tumor tissues with the assistance of Lipofectamine 2000 transfection, while NP-TP53-shMYC can increase the expression level of TP53 gene in tumor tissues regardless of whether it is assisted by Lipofectamine 2000 transfection. It can increase 900 times relative to the negative control group with the assistance of Lipofectamine 2000 transfection, and the multiple of induced TP53 expression increase is much higher than that of NP-TP53 regulated by single gene.

As can be seen from B of FIG. 22, for the MYC gene, the NP-shMYC multi-gene nanovector can reduce the MYC gene expression level in tumor tissue by nearly 80% compared with the negative control, while NP-TP53 has no significant effect on the MYC gene expression level in tumor tissue. L-TP53-shMYC can only reduce the MYC gene expression level in tumor tissue with the assistance of Lipofectamine 2000 transfection delivery, while NP-TP53-shMYC can reduce the MYC gene expression level in tumor tissue regardless of whether there is Lipofectamine 2000 transfection assistance, and it can achieve 40% MYC gene silencing efficiency with the assistance of Lipofectamine 2000 transfection.

As can be seen from FIG. 23, the analysis results of Western blotting are consistent with the results of qPCR, both indicate that the bidirectional gene regulation strategy of overexpressing tumor suppressor genes and silencing proto-oncogenes mediated by multi-gene nanovectors can greatly enhance the anti-tumor effect of tumor-transplanted mice, and is far higher than the therapeutic effect of single gene regulation.

### Test Example 8: Biological safety evaluation of multi-gene nanovectors NP-TP53-BIM-PTEN and NP-TP53-shMYC

The safety evaluation of multi-gene nanovectors mainly included organ tissue toxicity detection and immunotoxicity detection, and the detection methods were HE staining and ELISA detection of different inflammation-related factors. The specific operation process was as follows:

### a) HE staining

After the mice were executed, their heart, liver, spleen, lung, kidney and tumor tissues were taken and first subjected to tissue embedding. The tissues were dehydrated step by step with ethanol solutions having different concentrations: 75%, 85%, 95%, 100% (I), 100% (II), 40 minutes for each level of ethanol; the tissues were then immersed in three xylene cylinders in sequence, 30 minutes for each cylinder; the tissues were soaked in three paraffin cylinders in sequence, 1 hour in the first cylinder, 1.5 hours in the second cylinder, and 2 hours in the third cylinder; the liquid paraffin was poured into the mold box, and the wax-soaked tissue block was placed flat on the bottom, with the cut surface facing downward. After the paraffin solidified, the embedding frame was removed, and the wax block was trimmed after it was completely cooled and hardened. The paraffin on the periphery of the tissue was retained moderately for slicing. The pre-cooled wax block was fixed on the paraffin slicer so that the cut surface of the wax block was parallel to the knife edge. The inclination angle of the knife was usually 15 degrees. The rotary propeller was turned to adjust the slice thickness to 3-4µm, and sliced into slices with uniform thickness. The brush was held in your left hand, and the slicer handle was turned with your right hand. After the slices were brought out, the slices were lifted up gently with the brush, and the tweezers were then used to gently tweeze the wax slice and put into a slide box with the front side facing up. The water temperature was about 40 °C. After the slices were flattened, they were picked up. When the slices were attached, the operator held one end of the slice in the left hand and vertically put it into water to attach the slice, and used tweezers in the right hand to help push it and attach onto two-thirds of the slide. After the slice was attached, it was dried slightly in air, placed on a slice baking machine for baking at a temperature of 60 °C for 1 hour, and then placed in an oven and roasted for 2 hours. The paraffin slices were put into xylene (I) for 15 minutes, xylene (II) for 15 minutes, anhydrous ethanol (I) for 10 minutes, anhydrous ethanol (II) for 10 minutes, 95% alcohol for 10 minutes, and 85% alcohol for 10 minutes in sequence, and dewaxed in a gradient manner. The paraffin sections were stained with hematoxylin for 5-10 minutes, rinsed with tap water, differentiated with 1% hydrochloric acid alcohol for a few seconds, rinsed with tap water, then returned to blue with a saturated aqueous solution of lithium carbonate for 1 minute, rinsed with running water for a few seconds, stained with eosin dye for a few seconds, and rinsed with running water. The dehydrated and sealed paraffin sections were placed in 75% ethanol for 2 minutes, 85% ethanol for 2 minutes, anhydrous ethanol (I) for 2 minutes, anhydrous ethanol (II) for 2 minutes, and xylene for 2 minutes to make them transparent. The sections were taken out of xylene and sealed with neutral gum. The HE-stained pictures were collected by using an optical microscope, with the cell nucleus in blue and the cytoplasm in red.

### b) Detection of inflammation-related factors with ELISA

Before executing a mouse by breaking its neck, the whole blood was collected from the vein behind the mouse's eye socket and placed in an anticoagulant EP tube, which was placed in a 4 °C refrigerator overnight. The next day, the blood was centrifuged at 4 °C for about 30 minutes (with a rotational speed of 2,000rpm), and the supernatant was collected for detection of the levels of interleukin-6 (IL-6), tumor necrosis factor (TNF-α), and interferon (IFN-α, IFN-β, IFN-γ) in serum by means of Enzyme-Linked Immunosorbent Assay (ELISA). The detection of mouse interleukin-6 (IL-6) was taken as an example, the steps for other cytokines were the same, specifically: the kit should be taken out of the refrigerated environment and equilibrated at room temperature for 15-30 minutes before use, and the sample should also be equilibrated at room temperature for 60 minutes before use. The standard substance wells and sample wells were arranged, and 50µL of standard substances with different concentrations were added into each of the standard substance wells. The blank wells (blank control wells did not add samples and enzyme-labeled reagents, and the rest of the steps were the same) and test sample wells were arranged respectively. Firstly, 40µL of sample diluent was added to the sample well to be tested on the Enzyme-linked immunosorbent assay coated plate, and 10µL of the sample to be tested (the final dilution degree of the sample was 5 times) was then added. When adding samples, the samples were added to the bottom of the wells in the ELISA plate and shaken gently to mix uniformly. 100µL of the enzyme labeling reagent was added to each well, except for the blank wells. After the plate was sealed with a sealing film and incubated at 37 °C for 60min, the 20-fold concentrated washing solution was diluted with 20-fold distilled water for later use. The sealing film was carefully removed, the liquid was discarded, subjected to spin-drying, each well was filled with scrubbing solution, subjected to standing still for 30s and then discarded, the process was repeated for 5 times and the wells were subjected to pat drying. 50µL of color developer A was initially added into each well, 50µL of color developer B was then added, gently shaken and mixed uniformly, and developed color at 37 °C in the dark for 15min. 50µL of stop solution was added to each well to stop the reaction (the blue color immediately turned yellow). Zero setting was performed by using the blank wells, and the absorbance (OD value) of each well was measured in sequence at a wavelength of 450nm, the measurement should be performed within 15min after addition of the stop solution. The concentration of the standard substance was used as the horizontal axis and the OD value was used as the vertical axis, a standard curve was drawn on the coordinate paper to detect the curve equation (four-parameter Logistic fitting) and correlation coefficient R² of each cytokine. According to the OD value of the sample, the corresponding concentration was found from the standard curve, and then multiplied by the dilution multiple (5 times), which was the actual concentration of the sample.

On the basis of verifying the anti-tumor effectiveness of the multi-gene nanovector, the present disclosure further evaluated the biosafety in two tumor-bearing mouse models. The two tumor-bearing mouse models were the MDA-MB-231 transplanted tumor-bearing mouse model (5-week female BALB/c nude mice) and the NCI-H1299 transplanted tumor-bearing mouse model (6-8-week male BALB/c nude mice) respectively. The delivery of the multi-gene nanovector was achieved by intratumor injection. It was detected whether the morphology of NP-TP53-shMYC and NP-TP53-BIM-PTEN in different organs (heart, liver, spleen, lung, kidney, tumor) of the mice was normal according to the HE staining section of the tissue, the Enzyme-Linked Immunosorbent Assay (ELISA) method was used to detect whether the immune inflammatory factors (IL-6, TNF-α, IFN-α, IFN-β and IFN-γ) in the blood were abnormal, such that the systemic organ toxicity and immunotoxicity risk of the multi-gene nanovector to the mice were evaluated.

The results are shown in FIG. 24, FIG. 25, FIG. 26 and FIG. 27.

As illustrated by FIG. 24, for the NP-TP53-shMYC multi-gene nanovector, on the 16th day of administration, after the mice were killed, the heart, liver, spleen, lung, kidney and tumor tissues were taken, and HE staining pictures were collected under a microscope, different colors (cell nucleus was blue, cytoplasm was red) were used to identify whether the tissue morphology had abnormal changes. The results showed that the organs (heart, liver, spleen, lung, kidney) of mice in all treatment groups were consistent with those in the control group, without obvious abnormalities and lesions.

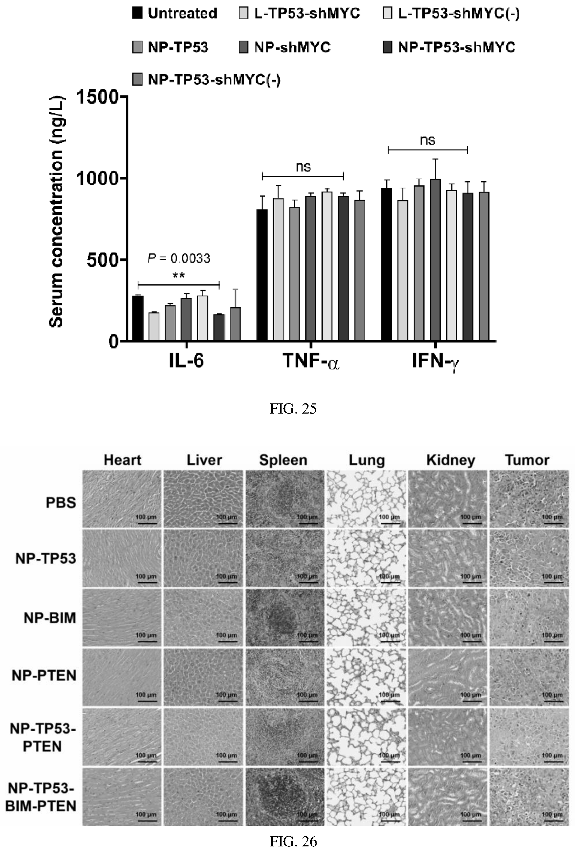

As illustrated by FIG. 25, 16 days after administration, before the mice were executed by breaking necks, whole blood was taken from the vein behind the mouse orbit, and the contents of three immune and cytokines (TNF-α, IFN-α and IL-6) in the mouse blood were detected by the ELISA kit. The contents of TNF-α, IFN-α and IL-6 in all treatment groups were close to those in the control group and maintained within the normal range.

The results indicated that NP-TP53-shMYC multi-gene nanovector had desirable biosafety.

As illustrated by FIG. 26, for the NP-TP53-BIM-PTEN multi-gene nanovector, on the 12th day of administration, after the mice were killed, the heart, liver, spleen, lung, kidney and tumor tissues were taken, and HE staining pictures were collected under a microscope, different colors (cell nucleus was blue, cytoplasm was red) were used to identify whether the tissue morphology had abnormal changes. The results showed that the organs (heart, liver, spleen, lung, kidney) of mice in all treatment groups were consistent with those in the control group, without obvious abnormalities and lesions.

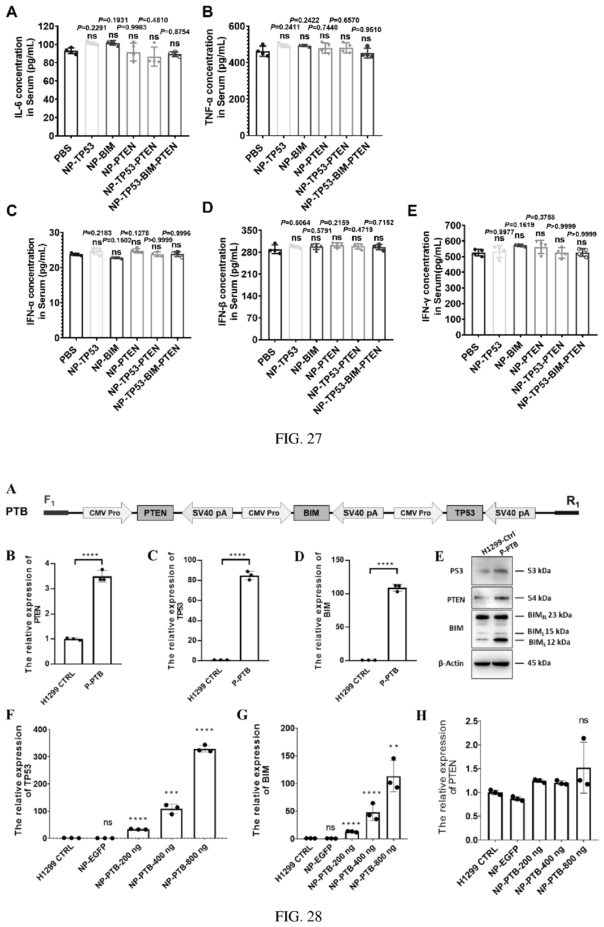

As illustrated by FIG. 27, 12 days after administration, before the mice were executed by breaking necks, whole blood was taken from the vein behind the mouse orbit, and the contents of three immune and cytokines (IL-6, TNF-α, IFN-α, IFN-β and IFN-γ) in the mouse blood were detected by the ELISA kit. The contents of IL-6, TNF-α, IFN-α, IFN-β and IFN-γ in all treatment groups were close to those in the control group and maintained within the normal range.

The results indicated that the NP-TP53-BIM-PTEN multi-gene nanovector had desirable biosafety.

### Test Example 9: Effects of different gene locations on co-expression of multiple genes in multi-gene nanovectors

In order to verify the influence of the position of the expression elements of three genes TP53, BIM and PTEN in the vector on the co-expression of the three genes, the present disclosure selected the NCI-H1299 non-small cell lung cancer cell line to test the feasibility of NP-PTEN-TP53-BIM in overexpression of three tumor suppressor genes in cancer cells. The co-expression conditions of plasmids P-PTEN-TP53-BIM (P-PTB) and NP-PTEN-TP53-BIM (NP-PTB) in NCI-H1299 cells were tested by using the transfection reagent Lipofectamine 3000.

The results are shown in FIG. 28, wherein A shows a schematic diagram of the PTEN-TP53-BIM (PTB) sequence; B illustrates the mRNA expression level of PTEN detected in P-PTB transfected cells by qPCR; C shows the mRNA expression level of TP53 detected in P-PTB transfected cells by qPCR; D illustrates the mRNA expression level of BIM detected in P-PTB transfected cells by qPCR; E shows the protein expression levels of PTEN, TP53, and BIM detected in P-PTB transfected cells by western blotting; F illustrates the dose-dependent expression analysis of NP-PTB, using qPCR to detect the mRNA expression level of TP53 in cells transfected with different doses of NP-PTB; G shows the dose-dependent expression analysis of NP-PTB, using qPCR to detect the mRNA expression level of BIM in cells transfected with different doses of NP-PTB. H illustrates the dose-dependent expression analysis of NP-PTB, using qPCR to detect the mRNA expression level of PTEN in cells transfected with different doses of NP-PTB.

As illustrated by FIG. 28, the qPCR and western blotting confirmed that P-PTB can simultaneously express TP53 and BIM genes, but cannot express PTEN gene. After preparation of the NP-PTB vector, NCI-H1299 was transfected with different doses (0-0.8 µg). As a negative control, the multi-gene nanovector NP-EGFP carrying the EGFP gene expression cassette could not induce the expression of TP53, BIM and PTEN after 48 hours of transfection. In cells transfected with NP-PTB, the mRNA of TP53 and BIM increased in a dose-dependent manner, but there was no significant increase in the induced expression of PTEN compared with the control group.

The results indicate that the NP-PTEN-TP53-BIM vector does not have the ability to express three genes synchronously, and the locations of the three gene expression elements are critical consideration factors in vector design.

### Test Example 10: Effects of repetitive promoters and transcription terminators on co-expression of multiple genes in multi-gene nanovectors

The present disclosure aimed to verify the effect of the common use of the same promoter and transcription terminator by the expression elements of three gene TP53, BIM and PTEN on the co-expression of the three genes, the NCI-H1299 non-small cell lung cancer cell line was selected to test the feasibility of NP-TP53-BIM-PTEN-V (NP-TBP-V) in simultaneous overexpression of the three tumor suppressor genes in cancer cells.

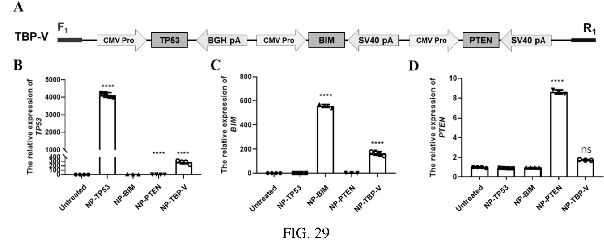

The results are shown in FIG. 29, wherein A shows a schematic diagram of the TP53-BIM-PTEN-V (TBP-V) sequence; B illustrates the detection of TP53 mRNA expression levels in NP-TBP-V transfected cells by using qPCR. C shows the detection of BIM mRNA expression levels in NP-TBP-V transfected cells by using qPCR. D illustrates the detection of PTEN mRNA expression levels in NP-TBP-V transfected cells by using the qPCR.

As can be seen from FIG. 29, qPCR confirmed that NP-TBP-V can express TP53 and BIM genes simultaneously, but still expresses PTEN at a low level, although the PTEN expression quantity of NP-TBP-V exceeds that of NP-PTB.

The results indicate that the NP-TBP-V vector does not have the ability to express three genes synchronously either, and different types of promoters and transcription terminators need to be used to ensure the co-expression ability of the three genes.

### Vector sequences

Linear DNA template L-TP53-BIM-PTEN
F1-[CMV-HA-TP53-BGH]-[SV40P-BIM-SV40pA]-[EF1a-FLAG-PTEN-WPRE-hGH pA]-R1 (sequence 25) 
1 CCTCCCAAGA TGGCCCGATA CGATGTACGG GCCAGATATA CGCGTTGACA
TTGATTATTG
61 ACTAGTTATT AATAGTAATC AATTACGGGG TCATTAGTTC ATAGCCCATA
TATGGAGTTC
121 CGCGTTACAT AACTTACGGT AAATGGCCCG CCTGGCTGAC CGCCCAACGA
CCCCCGCCCA
181 TTGACGTCAA TAATGACGTA TGTTCCCATA GTAACGCCAA TAGGGACTTT
CCATTGACGT
 
241 CAATGGGTGG AGTATTTACG GTAAACTGCC CACTTGGCAG TACATCAAGT
GTATCATATG
301 CCAAGTACGC CCCCTATTGA CGTCAATGAC GGTAAATGGC CCGCCTGGCA
TTATGCCCAG
361 TACATGACCT TATGGGACTT TCCTACTTGG CAGTACATCT ACGTATTAGT
CATCGCTATT
421 ACCATGGTGA TGCGGTTTTG GCAGTACATC AATGGGCGTG GATAGCGGTT
TGACTCACGG
481 GGATTTCCAA GTCTCCACCC CATTGACGTC AATGGGAGTT TGTTTTGGCA
CCAAAATCAA
541 CGGGACTTTC CAAAATGTCG TAACAACTCC GCCCCATTGA CGCAAATGGG
CGGTAGGCGT
601 GTACGGTGGG AGGTCTATAT AAGCAGAGCT CTCTGGCTAA CTAGAGAACC
CACTGCTTAC
661 TGGCTTATCG AAATTAATAC GACTCACTAT AGGGAGACCC AAGCTTACCA
TGGCCTACCC
721 CTACGACGTG CCCGACTACG CCTCCCTCGG ATCCGAGGAG CCGCAGTCAG
ATCCTAGCGT
781 CGAGCCCCCT CTGAGTCAGG AAACATTTTC AGACCTATGG AAACTACTTC
CTGAAAACAA
841 CGTTCTGTCC CCCTTGCCGT CCCAAGCAAT GGATGATTTG ATGCTGTCCC
CGGACGATAT
901 TGAACAATGG TTCACTGAAG ACCCAGGTCC AGATGAAGCT CCCAGAATGC
CAGAGGCTGC
961 TCCCCCCGTG GCCCCTGCAC CAGCAGCTCC TACACCGGCG GCCCCTGCAC
CAGCCCCCTC
1021 CTGGCCCCTG TCATCTTCTG TCCCTTCCCA GAAAACCTAC CAGGGCAGCT
ACGGTTTCCG
1081 TCTGGGCTTC TTGCATTCTG GGACAGCCAA GTCTGTGACT TGCACGTACT
CCCCTGCCCT
1141 CAACAAGATG TTTTGCCAAC TGGCCAAGAC CTGCCCTGTG CAGCTGTGGG
TTGATTCCAC
1201 ACCCCCGCCC GGCACCCGCG TCCGCGCCAT GGCCATCTAC AAGCAGTCAC
AGCACATGAC
1261 GGAGGTTGTG AGGCGCTGCC CCCACCATGA GCGCTGCTCA GATAGCGATG
GTCTGGCCCC
1321 TCCTCAGCAT CTTATCCGAG TGGAAGGAAA TTTGCGTGTG GAGTATTTGG
ATGACAGAAA
1381 CACTTTTCGA CATAGTGTGG TGGTGCCCTA TGAGCCGCCT GAGGTTGGCT
CAGACTGTAC
1441 CACCATCCAC TACAACTACA TGTGTAACAG TTCCTGCATG GGCGGCATGA
ACCGGAGGCC
1501 CATCCTCACC ATCATCACAC TGGAAGACTC CAGTGGTAAT CTACTGGGAC
GGAACAGCTT
1561 TGAGGTGCGT GTTTGTGCCT GTCCTGGGAG AGACCGGCGC ACAGAGGAAG
AGAATCTCCG
1621 CAAGAAAGGG GAGCCTCACC ACGAGCTGCC CCCAGGGAGC ACTAAGCGAG
CACTGCCCAA
1681 CAACACCAGC TCCTCTCCCC AGCCAAAGAA GAAACCACTG GATGGAGAAT
ATTTCACCCT
1741 TCAGATCCGT GGGCGTGAGC GCTTCGAGAT GTTCCGAGAG CTGAATGAGG
CCTTGGAACT
1801 CAAGGATGCC CAGGCTGGGA AGGAGCCAGG GGGGAGCAGG GCTCACTCCA
GCCACCTGAA
1861 GTCCAAAAAG GGTCAGTCTA CCTCCCGCCA TAAAAAACTC ATGTTCAAGA
CAGAAGGGCC
1921 TGACTCAGAC TGAGAATTCT GCAGATATCC ATCACACTGG CGGCCGCTCG
 
AGCATGCATC
1981 TAGAGGGCCC TATTCTATAG TGTCACCTAA ATGCTAGAGC TCGCTGATCA
GCCTCGACTG
2041 TGCCTTCTAG TTGCCAGCCA TCTGTTGTTT GCCCCTCCCC CGTGCCTTCC
TTGACCCTGG
2101 AAGGTGCCAC TCCCACTGTC CTTTCCTAAT AAAATGAGGA AATTGCATCG
CATTGTCTGA
2161 GTAGGTGTCA TTCTATTCTG GGGGGTGGGG TGGGGCAGGA CAGCAAGGGG
GAGGATTGGG
2221 AAGACAATAG CAGGCATGCT GGGGATGCGG TGGGCTCTAT GGCTTCTGAG
GCGGAAAGAA
2281 CCAGCTGGGG CTCTAGGGGG TATCCCCACG CGCCCTGTAG CGGCGCATTA
AGCGCGGCGA
2341 GCACCATGGC CTGAAATAAC CTCTGAAAGA GGAACTTGGT TAGGTACCTT
CTGAGGCGGA
2401 AAGAACCAGC TGTGGAATGT GTGTCAGTTA GGGTGTGGAA AGTCCCCAGG
CTCCCCAGCA
2461 GGCAGAAGTA TGCAAAGCAT GCATCTCAAT TAGTCAGCAA CCAGGTGTGG
AAAGTCCCCA
2521 GGCTCCCCAG CAGGCAGAAG TATGCAAAGC ATGCATCTCA ATTAGTCAGC
AACCATAGTC
2581 CCGCCCCTAA CTCCGCCCAT CCCGCCCCTA ACTCCGCCCA GTTCCGCCCA
TTCTCCGCCC
2641 CATGGCTGAC TAATTTTTTT TATTTATGCA GAGGCCGAGG CCGCCTCGGC
CTCTGAGCTA
2701 TTCCAGAAGT AGTGAGGAGG CTTTTTTGGA GGCCTAGGCT TTTGCAAAAA
GCTTGGCAAT
2761 CCGGTACTGT TGGTAAAGCC ACCATGGCAA AGCAACCTTC TGATGTAAGT
TCTGAGTGTG
2821 ACCGAGAAGG TAGACAATTG CAGCCTGCGG AGAGGCCTCC CCAGCTCAGA
CCTGGGGCCC
2881 CTACCTCCCT ACAGACAGAG CCACAAGCTT CCATGAGGCA GGCTGAACCT
GCAGATATGC
2941 GCCCAGAGAT ATGGATCGCC CAAGAGTTGC GGCGTATTGG AGACGAGTTT
AACGCTTACT
3001 ATGCAAGGAG GGTATTTTTG AATAATTACC AAGCAGCCGA AGACCACCCA
CGAATGGTTA
3061 TCTTACGACT GTTACGTTAC ATTGTCCGCC TGGTGTGGAG AATGCATTGA
TAATTCTAGA
3121 GTCGGGGCGG CCGGCCGCTT CGAGCAGACA TGATAAGATA CATTGATGAG
TTTGGACAAA
3181 CCACAACTAG AATGCAGTGA AAAAAATGCT TTATTTGTGA AATTTGTGAT
GCTATTGCTT
3241 TATTTGTAAC CATTATAAGC TGCAATAAAC AAGTTAACAA CAACAATTGC
ATTCATTTTA
3301 TGTTTCAGGT TCAGGGGGAG GTGTGGGAGG TTTTTTAAAG CAAGTAAAAC
CTCTACAAAT
3361 GTGGTAGGCT CCGGTGCCCG TCAGTGGGCA GAGCGCACAT CGCCCACAGT
CCCCGAGAAG
3421 TTGGGGGGAG GGGTCGGCAA TTGAACCGGT GCCTAGAGAA GGTGGCGCGG
GGTAAACTGG
3481 GAAAGTGATG TCGTGTACTG GCTCCGCCTT TTTCCCGAGG GTGGGGGAGA
ACCGTATATA
3541 AGTGCAGTAG TCGCCGTGAA CGTTCTTTTT CGCAACGGGT TTGCCGCCAG
AACACAGGTA
3601 AGTGCCGTGT GTGGTTCCCG CGGGCCTGGC CTCTTTACGG GTTATGGCCC
TTGCGTGCCT
 
3661 TGAATTACTT CCACCTGGCT GCAGTACGTG ATTCTTGATC CCGAGCTTCG
GGTTGGAAGT
3721 GGGTGGGAGA GTTCGAGGCC TTGCGCTTAA GGAGCCCCTT CGCCTCGTGC
TTGAGTTGAG
3781 GCCTGGCCTG GGCGCTGGGG CCGCCGCGTG CGAATCTGGT GGCACCTTCG
CGCCTGTCTC
3841 GCTGCTTTCG ATAAGTCTCT AGCCATTTAA AATTTTTGAT GACCTGCTGC
GACGCTTTTT
3901 TTCTGGCAAG ATAGTCTTGT AAATGCGGGC CAAGATCTGC ACACTGGTAT
TTCGGTTTTT
3961 GGGGCCGCGG GCGGCGACGG GGCCCGTGCG TCCCAGCGCA CATGTTCGGC
GAGGCGGGGC
4021 CTGCGAGCGC GGCCACCGAG AATCGGACGG GGGTAGTCTC AAGCTGGCCG
GCCTGCTCTG
4081 GTGCCTGGCC TCGCGCCGCC GTGTATCGCC CCGCCCTGGG CGGCAAGGCT
GGCCCGGTCG
4141 GCACCAGTTG CGTGAGCGGA AAGATGGCCG CTTCCCGGCC CTGCTGCAGG
GAGCTCAAAA
4201 TGGAGGACGC GGCGCTCGGG AGAGCGGGCG GGTGAGTCAC CCACACAAAG
GAAAAGGGCC
4261 TTTCCGTCCT CAGCCGTCGC TTCATGTGAC TCCACGGAGT ACCGGGCGCC
GTCCAGGCAC
4321 CTCGATTAGT TCTCGAGCTT TTGGAGTACG TCGTCTTTAG GTTGGGGGGA
GGGGTTTTAT
4381 GCGATGGAGT TTCCCCACAC TGAGTGGGTG GAGACTGAAG TTAGGCCAGC
TTGGCACTTG
4441 ATGTAATTCT CCTTGGAATT TGCCCTTTTT GAGTTTGGAT CTTGGTTCAT
TCTCAAGCCT
4501 CAGACAGTGG TTCAAAGTTT TTTTCTTCCA TTTCAGGTGT CGTGACGTAC
GGCCACCATG
4561 GACTACAAAG ACCATGACGG TGATTATAAA GATCATGACA TCGACTACAA
GGATGACGAT
4621 GACAAGCTTG GTACCGAGCT CGGATCCATG ACAGCCATCA TCAAAGAGAT
CGTTAGCAGA
4681 AACAAAAGGA GATATCAAGA GGATGGATTC GACTTAGACT TGACCTATAT
TTATCCAAAC
4741 ATTATTGCTA TGGGATTTCC TGCAGAAAGA CTTGAAGGCG TATACAGGAA
CAATATTGAT
4801 GATGTAGTAA GGTTTTTGGA TTCAAAGCAT AAAAACCATT ACAAGATATA
CAATCTTTGT
4861 GCTGAAAGAC ATTATGACAC CGCCAAATTT AATTGCAGAG TTGCACAATA
TCCTTTTGAA
4921 GACCATAACC CACCACAGCT AGAACTTATC AAACCCTTTT GTGAAGATCT
TGACCAATGG
4981 CTAAGTGAAG ATGACAATCA TGTTGCAGCA ATTCACTGTA AAGCTGGAAA
GGGACGAACT
5041 GGTGTAATGA TATGTGCATA TTTATTACAT CGGGGCAAAT TTTTAAAGGC
ACAAGAGGCC
5101 CTAGATTTCT ATGGGGAAGT AAGGACCAGA GACAAAAAGG GAGTAACTAT
TCCCAGTCAG
5161 AGGCGCTATG TGTATTATTA TAGCTACCTG TTAAAGAATC ATCTGGATTA
TAGACCAGTG
5221 GCACTGTTGT TTCACAAGAT GATGTTTGAA ACTATTCCAA TGTTCAGTGG
CGGAACTTGC
5281 AATCCTCAGT TTGTGGTCTG CCAGCTAAAG GTGAAGATAT ATTCCTCCAA
TTCAGGACCC
5341 ACACGACGGG AAGACAAGTT CATGTACTTT GAGTTCCCTC AGCCGTTACC
 
TGTGTGTGGT
5401 GATATCAAAG TAGAGTTCTT CCACAAACAG AACAAGATGC TAAAAAAGGA
CAAAATGTTT
5461 CACTTTTGGG TAAATACATT CTTCATACCA GGACCAGAGG AAACCTCAGA
AAAAGTAGAA
5521 AATGGAAGTC TATGTGATCA AGAAATCGAT AGCATTTGCA GTATAGAGCG
TGCAGATAAT
5581 GACAAGGAAT ATCTAGTACT TACTTTAACA AAAAATGATC TTGACAAAGC
AAATAAAGAC
5641 AAAGCCAACC GATACTTTTC TCCAAATTTT AAGGTGAAGC TGTACTTCAC
AAAAACAGTA
5701 GAGGAGCCGT CAAATCCAGA GGCTAGCAGT TCAACTTCTG TAACACCAGA
TGTTAGTGAC
5761 AATGAACCTG ATCATTATAG ATATTCTGAC ACCACTGACT CTGATCCAGA
GAATGAACCT
5821 TTTGATGAAG ATCAGCATAC ACAAATTACA AAAGTCTGAG AATTCGATAT
CAAGCTTATC
5881 GATAATCAAC CTCTGGATTA CAAAATTTGT GAAAGATTGA CTGGTATTCT
TAACTATGTT
5941 GCTCCTTTTA CGCTATGTGG ATACGCTGCT TTAATGCCTT TGTATCATGC
TATTGCTTCC
6001 CGTATGGCTT TCATTTTCTC CTCCTTGTAT AAATCCTGGT TGCTGTCTCT
TTATGAGGAG
6061 TTGTGGCCCG TTGTCAGGCA ACGTGGCGTG GTGTGCACTG TGTTTGCTGA
CGCAACCCCC
6121 ACTGGTTGGG GCATTGCCAC CACCTGTCAG CTCCTTTCCG GAACTTTCGC
TTTCCCCCTC
6181 CCTATTGCCA CGGCGGAACT CATCGCCGCC TGCCTTGCCC GCTGCTGGAC
AGGGGCTCGG
6241 CTGTTGGGCA CTGACAATTC CGTGGTGTTG TCGGGGAAAT CATCGTCCTT
TCCTTGGCTG
6301 CTCGCCTATG TTGCCACCTG GATTCTGCGC GGGACGTCCT TCTGCTACGT
CCCTTCGGCC
6361 CTCAATCCAG CGGACCTTCC TTCCCGCGGC CTGCTGCCGG CTCTGCGGCC
TCTTCCGCGT
6421 CTTCGCCTTC GCCCTCAGAC GAGTCGGATC TCCCTTTGGG CCGCCTCCCC
GCATCGATAC
6481 CGAGCGCTGC TCGAGAGATC TACGGGTGGC ATCCCTGTGA CCCCTCCCCA
GTGCCTCTCC
6541 TGGCCCTGGA AGTTGCCACT CCAGTGCCCA CCAGCCTTGT CCTAATAAAA
TTAAGTTGCA
6601 TCATTTTGTC TGACTAGGTG TCCTTCTATA ATATTATGGG GTGGAGGGGG
GTGGTATGGA
6661 GCAAGGGGCA AGTTGGGAAG ACAACCTGTA GGGCCTGCGG GGTCTATTGG
GAACCAAGCT
6721 GGAGTGCAGT GGCACAATCT TGGCTCACTG CAATCTCCGC CTCCTGGGTT
CAAGCGATTC
6781 TCCTGCCTCA GCCTCCCGAG TTGTTGGGAT TCCAGGCATG CATGACCAGG
CTCAGCTAAT
6841 TTTTGTTTTT TTGGTAGAGA CGGGGTTTCA CCATATTGGC CAGGCTGGTC
TCCAACTCCT
6901 AATCTCAGGT GATCTACCCA CCTTGGCCTC
CCAAATTGCT GGGATTACAG GCGTGAACCA
6961 CTGCTCCCTT CCCTGTCCTT TTTGGACAGG GAAGAGGAGG
Linear DNA template L-TP53-shmyC
F1-[CMV-TP53-BGH]-[hU6-shMYC-PolyT]-R1 (sequence 26)

1 CCTCCCAAGA TGGCCCGATA AGCTACAACA AGGCAAGGCT TGACCGACAA
TTGCATGAAG
61 AATCTGCTTA GGGTTAGGCG TTTTGCGCTG CTTCGCGATG TACGGGCCAG
ATATACGCGT
121 TGACATTGAT TATTGACTAG TTATTAATAG TAATCAATTA CGGGGTCATT
AGTTCATAGC
181 CCATATATGG AGTTCCGCGT TACATAACTT ACGGTAAATG GCCCGCCTGG
CTGACCGCCC
241 AACGACCCCC GCCCATTGAC GTCAATAATG ACGTATGTTC CCATAGTAAC
GCCAATAGGG
301 ACTTTCCATT GACGTCAATG GGTGGAGTAT TTACGGTAAA CTGCCCACTT
GGCAGTACAT
361 CAAGTGTATC ATATGCCAAG TACGCCCCCT ATTGACGTCA ATGACGGTAA
ATGGCCCGCC
421 TGGCATTATG CCCAGTACAT GACCTTATGG GACTTTCCTA CTTGGCAGTA
CATCTACGTA
481 TTAGTCATCG CTATTACCAT GGTGATGCGG TTTTGGCAGT ACATCAATGG
GCGTGGATAG
541 CGGTTTGACT CACGGGGATT TCCAAGTCTC CACCCCATTG ACGTCAATGG
GAGTTTGTTT
601 TGGCACCAAA ATCAACGGGA CTTTCCAAAA TGTCGTAACA ACTCCGCCCC
ATTGACGCAA
661 ATGGGCGGTA GGCGTGTACG GTGGGAGGTC TATATAAGCA GAGCTCTCTG
GCTAACTAGA
721 GAACCCACTG CTTACTGGCT TATCGAAATT AATACGACTC ACTATAGGGA
GACCCAAGCT
781 TACCATGGCC TACCCCTACG ACGTGCCCGA CTACGCCTCC CTCGGATCCG
AGGAGCCGCA
841 GTCAGATCCT AGCGTCGAGC CCCCTCTGAG TCAGGAAACA TTTTCAGACC
TATGGAAACT
901 ACTTCCTGAA AACAACGTTC TGTCCCCCTT GCCGTCCCAA GCAATGGATG
ATTTGATGCT
961 GTCCCCGGAC GATATTGAAC AATGGTTCAC TGAAGACCCA GGTCCAGATG
AAGCTCCCAG
1021 AATGCCAGAG GCTGCTCCCC CCGTGGCCCC TGCACCAGCA GCTCCTACAC
CGGCGGCCCC
1081 TGCACCAGCC CCCTCCTGGC CCCTGTCATC TTCTGTCCCT TCCCAGAAAA
CCTACCAGGG
1141 CAGCTACGGT TTCCGTCTGG GCTTCTTGCA TTCTGGGACA GCCAAGTCTG
TGACTTGCAC
1201 GTACTCCCCT GCCCTCAACA AGATGTTTTG CCAACTGGCC AAGACCTGCC
CTGTGCAGCT
1261 GTGGGTTGAT TCCACACCCC CGCCCGGCAC CCGCGTCCGC GCCATGGCCA
TCTACAAGCA
1321 GTCACAGCAC ATGACGGAGG TTGTGAGGCG CTGCCCCCAC CATGAGCGCT
GCTCAGATAG
1381 CGATGGTCTG GCCCCTCCTC AGCATCTTAT CCGAGTGGAA GGAAATTTGC
GTGTGGAGTA
1441 TTTGGATGAC AGAAACACTT TTCGACATAG TGTGGTGGTG CCCTATGAGC
CGCCTGAGGT
1501 TGGCTCAGAC TGTACCACCA TCCACTACAA CTACATGTGT AACAGTTCCT
GCATGGGCGG
1561 CATGAACCGG AGGCCCATCC TCACCATCAT CACACTGGAA GACTCCAGTG

GTAATCTACT
1621 GGGACGGAAC AGCTTTGAGG TGCGTGTTTG TGCCTGTCCT GGGAGAGACC
GGCGCACAGA
1681 GGAAGAGAAT CTCCGCAAGA AAGGGGAGCC TCACCACGAG CTGCCCCCAG
GGAGCACTAA
1741 GCGAGCACTG CCCAACAACA CCAGCTCCTC TCCCCAGCCA AAGAAGAAAC
CACTGGATGG
1801 AGAATATTTC ACCCTTCAGA TCCGTGGGCG TGAGCGCTTC GAGATGTTCC
GAGAGCTGAA
1861 TGAGGCCTTG GAACTCAAGG ATGCCCAGGC TGGGAAGGAG CCAGGGGGGA
GCAGGGCTCA
1921 CTCCAGCCAC CTGAAGTCCA AAAAGGGTCA GTCTACCTCC CGCCATAAAA
AACTCATGTT
1981 CAAGACAGAA GGGCCTGACT CAGACTGAGA ATTCTGCAGA TATCCATCAC
ACTGGCGGCC
2041 GCTCGAGCAT GCATCTAGAG GGCCCTATTC TATAGTGTCA CCTAAATGCT
AGAGCTCGCT
2101 GATCAGCCTC GACTGTGCCT TCTAGTTGCC AGCCATCTGT TGTTTGCCCC
TCCCCCGTGC
2161 CTTCCTTGAC CCTGGAAGGT GCCACTCCCA CTGTCCTTTC CTAATAAAAT
GAGGAAATTG
2221 CATCGCATTG TCTGAGTAGG TGTCATTCTA TTCTGGGGGG TGGGGTGGGG
CAGGACAGCA
2281 AGGGGGAGGA TTGGGAAGAC AATAGCAGGC ATGCTGGGGA TGCGGTGGGC
TCTATGGCTT
2341 CTGAGGCGGA AAGAACCAGC TGGGGCTCTA GGGGGTATCC CCACGCGCCC
TGTAGCGGCG
2401 CATTAAGCGC GAGGGCCTAT TTCCCATGAT TCCTTCATAT TTGCATATAC
GATACAAGGC
2461 TGTTAGAGAG ATAATTGGAA TTAATTTGAC TGTAAACACA AAGATATTAG
TACAAAATAC
2521 GTGACGTAGA AAGTAATAAT TTCTTGGGTA GTTTGCAGTT TTAAAATTAT
GTTTTAAAAT
2581 GGACTATCAT ATGCTTACCG TAACTTGAAA GTATTTCGAT TTCTTGGCTT
TATATATCTT
2641 GTGGAAAGGA CGAAACACCG CTTCACCAAC AGGAACTATG CGAACATAGT
TCCTGTTGGT
2701 GAAGCCACAG TTCGGTAAGG GAGAGAGAAT GTCAAGAGGC GAACACGAAT
GTTCGCCTCT
2761 TGACATTCTC CACAGTTCGG TAAGGGAGAG CTCATTTCTG AAGAGGACTT
CGAAAAGTCC
2821 TCTTCAGAAA TGAGCCACAG TTCGGTAAGG GAGAGGAAAC GACGAGAACA
GTTGACGAAT
2881 CAACTGTTCT CGTCGTTTCC TTTTTTCTCG AGTTTGGACA GGGAAGAGGA GG

The above content describes in detail the preferred embodiments of the present disclosure, but the present disclosure is not limited thereto. A variety of simple modifications can be made in regard to the technical solutions of the present disclosure within the scope of the technical concept of the present disclosure, including a combination of individual technical features in any other suitable manner, such simple modifications and combinations thereof shall also be regarded as the content disclosed by the present disclosure, each of them falls into the protection scope of the present disclosure.

## Claims

1. A multi-gene nanovector, **characterized in that** the multi-gene nanovector comprises a three-branch central ligand and gene combination sequences covalently connected on each branch of the three-branch central ligand, the gene combination sequences on each branch are the same; the gene combination sequences comprise at least two tumor suppressor genes selected from TP53, BIM and PTEN successively in the direction extending outward from the three-branch central ligand.

2. The multi-gene nanovector according to claim 1, **characterized in that** terminals of the gene combination sequences further comprise a proto-oncogene MYC in the direction extending outward from the three-branch central ligand.

3. The multi-gene nanovector according to claim 1, **characterized in that** the gene combination sequences comprise three tumor suppressor genes TP53, BIM and PTEN successively in the direction extending outward from the three-branch central ligand.

4. A multi-gene nanovector, **characterized in that** the multi-gene nanovector comprises a three-branch central ligand and gene combination sequences covalently connected on each branch of the three-branch central ligand, the gene combination sequences on each branch are the same; the gene combination sequences comprise tumor suppressor genes and a proto-oncogene successively in the direction extending outward from the three-branch central ligand, wherein the tumor suppressor genes is one selected from the group consisting of TP53, BIM and PTEN, and the proto-oncogene is MYC.

5. The multi-gene nanovector according to claim 4, **characterized in that** the gene combination sequences comprise TP53 and MYC successively in the direction extending outward from the three-branch central ligand.

6. The multi-gene nanovector according to any one of claims 1-5, **characterized in that** the three-branch central ligand is provided by a three-branch central compound represented by formula (I);

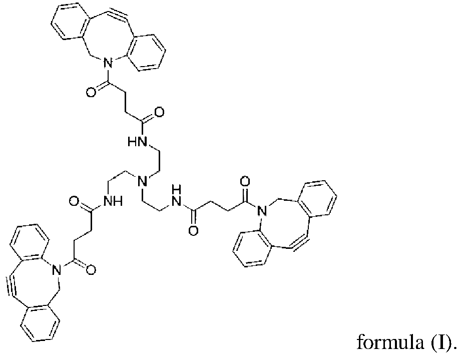

7. A method for preparing the multi-gene nanovector according to any one of claims 1-6, **characterized in that** the method comprises the following steps:
(1) performing a Click cross-linking reaction on the three-branch central compound represented by formula (I) and a single-stranded primer nucleic acid with an azide modification at the 5' end to obtain a tri-branched primer containing 3 primers;
(2) carrying out a branch PCR reaction in a PCR reaction system by using the tri-branched primer as a primer and using a linear DNA as a template to obtain the multi-gene nanovector; wherein the sequence information of the linear DNA is consistent with the gene combination sequences in the multi-gene nanovector according to any one of claims 1-5;

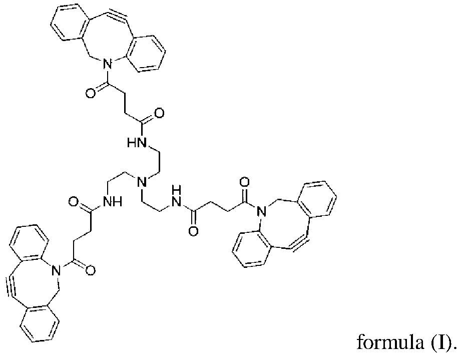

8. A use of the multi-gene nanovector according to any one of claims 1-6 in the preparation of an anti-cancer drug.

9. The use according to claim 8, **characterized in that** the anti-cancer drug is an anti-breast cancer drug and/or an anti-lung cancer drug.

10. An anti-cancer drug, **characterized in that** the anti-cancer drug consists of an active ingredient and an adjuvant, wherein the active ingredient comprises the multi-gene nanovector according to any one of claims 1-6.

11. The anti-cancer drug according to claim 10, **characterized in that** the content of the active ingredient is within the range of 0.01-99.99wt%.

12. The anti-cancer drug according to claim 10 or 11, **characterized in that** the adjuvant comprises liposome 2000 and/or liposome 3000.
